# FASCICULE DE BREVET EUROPEEN

(11) **EP 1 228 193 B1**
(45) Date de publication et mention de la délivrance du brevet: **16.01.2008**
(21) Numéro de dépôt: 00953243.3
(22) Date de dépôt: 13.07.2000
(51) Int. Cl.: C12N 1/00

(54) **GENES DE CALICIVIRUS FELIN ET VACCINS RECOMBINES LES CONTENANT**
GENE VON FELINCALIZIVIRUS UND IMPSTOFFE, DIE DIESE GENE ENTHALTEN
FELINE CALICIVIRUS GENES AND VACCINES, IN PARTICULAR RECOMBINED VACCINES

(30) Priorité: 16.07.1999 FR 9909421; 11.02.2000 FR 0001761
(43) Date de publication de la demande: 07.08.2002
(73) Titulaire: MERIAL, 69002 Lyon (FR)
(72) Inventeur: AUDONNET, Jean-Christophe, Francis, F-69006 Lyon (FR); BAUDU, Philippe, Guy, Nicolas, F-69290 Craponne (FR); BRUNET, Sylvie, Claudine, F-69005 Lyon (FR)
(74) Mandataire: Nargolwalla, Cyra
(86) Numéro de dépôt international: PCT/FR2000/002051
(87) Numéro de publication internationale: WO 2001/005934

(56) Documents cités:
- EP-A- 0 737 750
- EP-A- 0 786 518
- WO-A-91/01332
- WO-A-94/16716
- WO-A-96/39177
- WO-A-98/03660
- WO-A-98/21354
- WO-A-98/56929
- US-A- 5 858 373
- TARTAGLIA J ET AL: "PROTECTION OF CATS AGAINST FELINE LEUKEMIA VIRUS BY VACCINATION WITH A CANARYPOX VIRUS RECOMBINANT, ALVAC-FL" JOURNAL OF VIROLOGY,US,NEW YORK, US, vol. 67, no. 4, avril 1993 (1993-04), pages 2370-2375, XP002916084 ISSN: 0022-538X
- PAOLETTI ENZO: "Applications of pox virus vectors to vaccination: An update." PROCEEDINGS OF THE NATIONAL ACADEMY OF SCIENCES OF THE UNITED STATES OF AMERICA 1996, vol. 93, no. 21, 1996, pages 11349-11353, XP002135943 ISSN: 0027-8424
- POULET H ET AL: "COMPARISON BETWEEN ACUTE ORAL/RESPIRATORY AND CHRONIC STOMATITIS/GINGIVITIS ISOLATES OF FELINE CALICIVIRUS: PATHOGENICITY, ANTIGENIC PROFILE AND CROSS-NEUTRALISATION STUDIES" ARCHIVES OF VIROLOGY,US,NEW YORK, NY, vol. 145, no. 2, 2000, pages 243-261, XP000914073 ISSN: 0304-8608

### LISTE DE SEQUENCES

<110> MERIAL
<120> Gènes de calicivirus félin et vaccin recombiné les incorporant
<130> FCVrecombiné
<140>
   <141> 2000-02-11
<160> 23
<170> PatentIn Ver. 2.1
<210> 1
   <211> 33
   <212> ADN
   <213> Séquence artificielle
<220>
   <223> Description de la séquence artificielle: oligonucleotide
<400> 1
   ttgcggccgc tgtgatgtgt tcgaagtttg agc 33
<210> 2
   <211> 36
   <212> ADN
   <213> Séquence artificielle
<220>
   <223> Description de la séquence artificielle: oligonucleotide
<400> 2
   ttggcgccgy tgaccmagtg cagcyttrtc caattc 36
<210> 3
   <211> 38
   <212> ADN
   <213> Séquence artificielle
<220>
   <223> Description de la séquence artificielle: oligonucleotide
<400> 3
   ttggcgccaa ywgtrttwgh tacagtrtca acyarrcc 38
<210> 4
   <211> 2010
   <212> ADN
   <213> calicivirus félin
<400> 4
   atgtgctcaa cctgcgctaa cgtgcttaaa tactatgatt gggatcccca cacaaaattg 60
   gttattgacc ccaataaatt cctttctcta ggcttctgcg ataaaccgct tttatgctgc 120
   tacccagaac ttctcccaga atttggaaca gtgtgggatt gtgaccaatc ccctctacaa 180
   atttaccttg aatctatcct tggtgatgat gaatggagct cgacatttga tgctatcgat 240
   cctgttgttc ctcccatgca ttgggacaag gctgggaaaa tcttccagcc tcatcctggt 300
   gttctaatgc accacctcat caatgaagtt gcaaaagctt gggatccaaa tctccccatc 360
   ttccgattgg aagctgacgg ggattcatcc atcacgaccc ctgagcaagg aacattggtc 420
   ggtggtgtta ttgccgagcc cagcgctcaa atggcaactg ctgctgacgc agcaactggc 480
   aagagtgttg actcggaatc ggagtctttc ttctcattcc atactagtgt gaattggagt 540
   acatctgaaa cccagggaaa gatcctcttt aaacaatctt taggacccct acttaatcct 600
   taccttgaac acctttctaa attatacgtt gcttggtctg gatcagtgga tgtaaggttc 660
   tctatttctg gctccggtgt cttcgggggg aaattggctg ccattgttgt gcctccaggg 720
   gttgaccccg tccagagcac gtcaatgctc cagtatcccc atgtcctctt tgatgctcgc 780
   caagttgaac ctgttatatt ttcaatcccc gatttaagga gcactctcta tcacctaatg 840
   tctgatactg atactacatc ccttgttatc atggtatata atgatcttat taacccttat 900
   gctaatgatt ccaactcttc tgggtgtatt gttaccgttg agaccaaacc tggacctgac 960
   ttcaaatttc acctcctgaa accacctgga tcaatgttaa ctcatggctc tattccctct 1020
   gacttgattc caaaatcttc atccctttgg attggaaatc gatattggtc tgacataact 1080
   gattttgtaa ttcggccatt cgtgtttcaa gccaatcgtc actttgactt caaccaagaa 1140
   acggctggat ggagcacacc aagatttcga cccataacaa taactattag tgaaagtaat 1200
   ggatcaaaac tgggaactgg cgtggccaca gattacattg tgcccggcat acctgatggt 1260
   tggcctgaca ccacaattgg tgaggaattg acaccagctg gagattactc aatcacaaac 1320
   ggtagtggca atgacattgc aacagctaat gcttatgaca gtgctgatgt gatcacaaac 1380
   accacaaatt tcagggggat gtacatttgt ggagcactcc agagggcttg gggcgataag 1440
   aagatctcaa gtacagcttt cataaccact gctattaagg aaggtaatac gcttaaacca 1500
   tcaaatacaa ttgacatgac aaaaattgct gtgtaccagg acactcatgt tggcagggat 1560
   gttcaaacat ctgatgatac actggcaatc cttggttaca ctggaattgg tgaacaggca 1620
   attggatcta atagggatag tgtggttcgc attagcatgc tgccggaaac tggtgcccgc 1680
   ggcgggaatc acccaatttt ctacaaaaat tctattaagt taggatatgt actcaggtca 1740
   attgatgtgt tcaactcaca aattctccac acatctagac aactgtccct caatcattac 1800
   ttgctaccac ctgactcatt tgctgtttat aggattatag actctaatgg atcttggttt 1860
   gatgtaggga ttgatagtga tggtttttcc tttgttggtg tttctagtat ccctaaactt 1920
   gagtttcctc tttctgcctc ctacatggga attcagctgg caaagattcg acttgcctct 1980
   aacattagga gtactatgac aaaactatga                                  2010
<210> 5
   <211> 669
   <212> PRT
   <213> calicivirus félin
<400> 5
   Met Cys Ser Thr Cys Ala Asn Val Leu Lys Tyr Tyr Asp Trp Asp Pro
   1 5 10 15
   His Thr Lys Leu Val Ile Asp Pro Asn Lys Phe Leu Ser Leu Gly Phe
   20 25 30
   Cys Asp Lys Pro Leu Leu Cys Cys Tyr Pro Glu Leu Leu Pro Glu Phe
   35 40 45
   Gly Thr Val Trp Asp Cys Asp Gln Ser Pro Leu Gln Ile Tyr Leu Glu
   50 55 60
   Ser Ile Leu Gly Asp Asp Glu Trp Ser Ser Thr Phe Asp Ala Ile Asp
   65 70 75 80
   Pro Val Val Pro Pro Met His Trp Asp Lys Ala Gly Lys Ile Phe Gln
   85 90 95
   Pro His Pro Gly Val Leu Met His His Leu Ile Asn Glu Val Ala Lys
   100 105 110
   Ala Trp Asp Pro Asn Leu Pro Ile Phe Arg Leu Glu Ala Asp Gly Asp
   115 120 125
   Ser Ser Ile Thr Thr Pro Glu Gln Gly Thr Leu Val Gly Gly Val Ile
   130 135 140
   Ala Glu Pro Ser Ala Gln Met Ala Thr Ala Ala Asp Ala Ala Thr Gly
   145 150 155 160
   Lys Ser Val Asp Ser Glu Trp Glu Ser Phe Phe Ser Phe His Thr Ser
   165 170 175
   Val Asn Trp Ser Thr Ser Glu Thr Gln Gly Lys Ile Leu Phe Lys Gln
   180 185 190
   Ser Leu Gly Pro Leu Leu Asn Pro Tyr Leu Glu His Leu Ser Lys Leu
   195 200 205
   Tyr Val Ala Trp Ser Gly Ser Val Asp Val Arg Phe Ser Ile Ser Gly
   210 215 220
   Ser Gly Val Phe Gly Gly Lys Leu Ala Ala Ile Val Val Pro Pro Gly
   225 230 235 240
   Val Asp Pro Val Gln Ser Thr Ser Met Leu Gln Tyr Pro His Val Leu
   245 250 255
   Phe Asp Ala Arg Gln Val Glu Pro Val Ile Phe Ser Ile Pro Asp Leu
   260 265 270
   Arg Ser Thr Leu Tyr His Leu Met Ser Asp Thr Asp Thr Thr Ser Leu
   275 280 285
   Val Ile Met Val Tyr Asn Asp Leu Ile Asn Pro Tyr Ala Asn Asp Ser
   290 295 300
   Asn Ser Ser Gly Cys Ile Val Thr Val Glu Thr Lys Pro Gly Pro Asp
   305 310 315 320
   Phe Lys Phe His Leu Leu Lys Pro Pro Gly Ser Met Leu Thr His Gly
   325 330 335
   Ser Ile Pro Ser Asp Leu Ile Pro Lys Ser Ser Ser Leu Trp Ile Gly
   340 345 350
   Asn Arg Tyr Trp Ser Asp Ile Thr Asp Phe Val Ile Arg Pro Phe Val
   355 360 365
   Phe Gln Ala Asn Arg His Phe Asp Phe Asn Gln Glu Thr Ala Gly Trp
   370 375 380
   Ser Thr Pro Arg Phe Arg Pro Ile Thr Ile Thr Ile Ser Glu Ser Asn
   385 390 395 400
   Gly Ser Lys Leu Gly Thr Gly Val Ala Thr Asp Tyr Ile Val Pro Gly
   405 410 415
   Ile Pro Asp Gly Trp Pro Asp Thr Thr Ile Gly Glu Glu Leu Thr Pro
   420 425 430
   Ala Gly Asp Tyr Ser Ile Thr Asn Gly Ser Gly Asn Asp Ile Ala Thr
   435 440 445
   Ala Asn Ala Tyr Asp Ser Ala Asp Val Ile Thr Asn Thr Thr Asn Phe
   450 455 460
   Arg Gly Met Tyr Ile Cys Gly Ala Leu Gln Arg Ala Trp Gly Asp Lys
   465 470 475 480
   Lys Ile Ser Ser Thr Ala Phe Ile Thr Thr Ala Ile Lys Glu Gly Asn
   485 490 495
   Thr Leu Lys Pro Ser Asn Thr Ile Asp Met Thr Lys Ile Ala Val Tyr
   500 505 510
   Gln Asp Thr His Val Gly Arg Asp Val Gln Thr Ser Asp Asp Thr Leu
   515 520 525
   Ala Ile Leu Gly Tyr Thr Gly Ile Gly Glu Gln Ala Ile Gly Ser Asn
   530 535 540
   Arg Asp Ser Val Val Arg Ile Ser Met Leu Pro Glu Thr Gly Ala Arg
   545 550 555 560
   Gly Gly Asn His Pro Ile Phe Tyr Lys Asn Ser Ile Lys Leu Gly Tyr
   565 570 575
   Val Leu Arg Ser Ile Asp Val Phe Asn Ser Gln Ile Leu His Thr Ser
   580 585 590
   Arg Gln Leu Ser Leu Asn His Tyr Leu Leu Pro Pro Asp Ser Phe Ala
   595 600 605
   Val Tyr Arg Ile Ile Asp Ser Asn Gly Ser Trp Phe Asp Val Gly Ile
   610 615 620
   Asp Ser Asp Gly Phe Ser Phe Val Gly Val Ser Ser Ile Pro Lys Leu
   625 630 635 640
   Glu Phe Pro Leu Ser Ala Ser Tyr Met Gly Ile Gln Leu Ala Lys Ile
   645 650 655
   Arg Leu Ala Ser Asn Ile Arg Ser Thr Met Thr Lys Leu
   660 665
<210> 6
   <211> 2007
   <212> ADN
   <213> calicivirus félin
<400> 6
   atgtgctcaa cctgcgctaa cgtgcttaaa tactatgatt gggatcccca ctttagattg 60
   attattaacc ccaacaaatt tctttccgtt ggcttctgtg ataatcctct tatgtgttgt 120
   tatcccgaat tactccctga atttggaact gtgtgggact gtgatcagtc accactccaa 180
   atttatctag agtccatcct tggtgatgac gaatgggctt ccacttacga agcagttgac 240
   ccagtggtgc caccaatgca ttgggatagt gctggaaaga tctttcagcc acatcctggt 300
   gtattgatgc accatctgat tggtgaagtt gctaaggcct gggatccaaa cttaccactc 360
   tttcgtctgg aagcggatga tggatctgtg accacgcctg aacaaggaac actggttggt 420
   ggagtcattg ctgagcctaa tgcccaaatg tcagctgttg ctgacgtggc cactggcaaa 480
   agtgttgact ctgagtggga agcattcttc tctttccaca ccagtgtcaa ttggagcaca 540
   tctgaaaccc aagggaaaat cctttttaaa caatctctag gtcccctact taacccttac 600
   cttactcatc tcgcaaaact ttatgttgca tggtctggtt ctattgaggt tagattttca 660
   atttctggat ctggtgtctt tggtggaaaa ctggctgcta ttgttgtgcc acccgggatc 720
   gatcccgtgc aaagcacatc aatgttgcag tacccccatg ttctgtttga tgctcgtcaa 780
   gttgaacctg ttatcttcac tatccctgat ttgagaaata gtctatatca ccttatgtct 840
   gacactgata ctacatctct tgtcattatg atatacaatg atctcattaa tccctatgct 900
   aatgattcta actcatctgg atgcattgtt actgtggaga caaaacctgg ccccgatttc 960
   aaatttcacc tcttgaaacc gcctgggtct atgttaactc atgggtcaat tccatccgac 1020
   cttatcccaa aatcttcttc tctttggatt ggcaaccgac actggtctga tataactgat 1080
   tttgtcatca aaccttttgt tttccaggct aatcgacatt ttgacttcaa tcaagagact 1140
   gcaggctgga gcactcccag atttagaccc ataaccatca cagtttctga gaagggagga 1200
   tcaaaattgg gtattggtgt tgcaactgac tctattgtcc ctggcatacc agacggctgg 1260
   ccggatacca ccattccaga aaaacttacc ccagcaggtg actatgcaat cacaaatggg 1320
   ggaaacaatg acatcaccac tgctgcggac tatgatgggg caagtataat caaaaacaat 1380
   acaaatttca agggtatgta tatttgtggt gctttgcaaa gagcttgggg tgacaagaaa 1440
   atttcaaaca ctgcctttat cactaccgca atcagagagg gtaactcaat aaaaccatct 1500
   aatgtaattg acatgacaaa acttgccgtt tatcaagatg ctcatgttgg tgcagaactt 1560
   caaacctctg acatcacctt agcaatcctt ggttataccg ggattggtga agaagctata 1620
   ggcctggata gggacaaagt ggtgcgtatt agcatacttc cagaaactgg tgctcgtggc 1680
   ggaaatcacc ctattttcta tatgaacaaa attaaattag gttatgttat tagatcaata 1740
   gatgttgcaa actcccaaat tttacataca tctaggcaat tatcactcaa taattatcta 1800
   ctggctcctg actcctttgc agtttacaga attattgatt ctggcggctc ttggtttgat 1860
   attgctattg atagtgatgg tttttctttt gttggtgtat ctcaaattgg aaaattggag 1920
   tttccactaa ctgcctccta catgggaatt caattggcaa agattcgact tgcctcaaac 1980
   attagcagtg gaatggttaa aatatga                                    2007
<210> 7
   <211> 668
   <212> PRT
   <213> calicivirus félin
<400> 7
   Met Cys Ser Thr Cys Ala Asn Val Leu Lys Tyr Tyr Asp Trp Asp Pro
   1 5 10 15
   His Phe Arg Leu Ile Ile Asn Pro Asn Lys Phe Leu Ser Val Gly Phe
   20 25 30
   Cys Asp Asn Pro Leu Met Cys Cys Tyr Pro Glu Leu Leu Pro Glu Phe
   35 40 45
   Gly Thr Val Trp Asp Cys Asp Gln Ser Pro Leu Gln Ile Tyr Leu Glu
   50 55 60
   Ser Ile Leu Gly Asp Asp Glu Trp Ala Ser Thr Tyr Glu Ala Val Asp
   65 70 75 80
   Pro Val Val Pro Pro Met His Trp Asp Ser Ala Gly Lys Ile Phe Gln
   85 90 95
   Pro His Pro Gly Val Leu Met His His Leu Ile Gly Glu Val Ala Lys
   100 105 110
   Ala Trp Asp Pro Asn Leu Pro Leu Phe Arg Leu Glu Ala Asp Asp Gly
   115 120 125
   Ser Val Thr Thr Pro Glu Gln Gly Thr Leu Val Gly Gly Val Ile Ala
   130 135 140
   Glu Pro Asn Ala Gln Met Ser Ala Val Ala Asp Val Ala Thr Gly Lys
   145                 150                 155                 160
   Ser Val Asp Ser Glu Trp Glu Ala Phe Phe Ser Phe His Thr Ser Val
                   165                 170                 175
   Asn Trp Ser Thr Ser Glu Thr Gln Gly Lys Ile Leu Phe Lys Gln Ser
               180                 185                 190
   Leu Gly Pro Leu Leu Asn Pro Tyr Leu Thr His Leu Ala Lys Leu Tyr
           195                 200                 205
   Val Ala Trp Ser Gly Ser Ile Glu Val Arg Phe Ser Ile Ser Gly Ser
       210                 215                 220
   Gly Val Phe Gly Gly Lys Leu Ala Ala Ile Val Val Pro Pro Gly Ile
   225                 230                 235                 240
   Asp Pro Val Gln Ser Thr Ser Met Leu Gln Tyr Pro His Val Leu Phe
                   245                 250                 255
   Asp Ala Arg Gln Val Glu Pro Val Ile Phe Thr Ile Pro Asp Leu Arg
               260                 265                 270
   Asn Ser Leu Tyr His Leu Met Ser Asp Thr Asp Thr Thr Ser Leu Val
           275                 280                 285
   Ile Met Ile Tyr Asn Asp Leu Ile Asn Pro Tyr Ala Asn Asp Ser Asn
       290                 295                 300
   Ser Ser Gly Cys Ile Val Thr Val Glu Thr Lys Pro Gly Pro Asp Phe
   305                 310                 315                 320
   Lys Phe His Leu Leu Lys Pro Pro Gly Ser Met Leu Thr His Gly Ser
                   325                 330                 335
   Ile Pro Ser Asp Leu Ile Pro Lys Ser Ser Ser Leu Trp Ile Gly Asn
               340                 345                 350
   Arg His Trp Ser Asp Ile Thr Asp Phe Val Ile Lys Pro Phe Val Phe
           355                 360                 365
   Gln Ala Asn Arg His Phe Asp Phe Asn Gln Glu Thr Ala Gly Trp Ser
       370                 375                 380
   Thr Pro Arg Phe Arg Pro Ile Thr Ile Thr Val Ser Glu Lys Gly Gly
   385                 390                 395                 400
   Ser Lys Leu Gly Ile Gly Val Ala Thr Asp Ser Ile Val Pro Gly Ile
                   405                 410                 415
   Pro Asp Gly Trp Pro Asp Thr Thr Ile Pro Glu Lys Leu Thr Pro Ala
               420                 425                 430
   Gly Asp Tyr Ala Ile Thr Asn Gly Gly Asn Asn Asp Ile Thr Thr Ala
           435                 440                 445
   Ala Asp Tyr Asp Gly Ala Ser Ile Ile Lys Asn Asn Thr Asn Phe Lys
       450                 455                 460
   Gly Met Tyr Ile Cys Gly Ala Leu Gln Arg Ala Trp Gly Asp Lys Lys
   465 470 475 480
   Ile Ser Asn Thr Ala Phe Ile Thr Thr Ala Ile Arg Glu Gly Asn Ser
   485 490 495
   Ile Lys Pro Ser Asn Val Ile Asp Met Thr Lys Leu Ala Val Tyr Gln
   500 505 510
   Asp Ala His Val Gly Ala Glu Leu Gln Thr Ser Asp Ile Thr Leu Ala
   515 520 525
   Ile Leu Gly Tyr Thr Gly Ile Gly Glu Glu Ala Ile Gly Leu Asp Arg
   530 535 540
   Asp Lys Val Val Arg Ile Ser Ile Leu Pro Glu Thr Gly Ala Arg Gly
   545 550 555 560
   Gly Asn His Pro Ile Phe Tyr Met Asn Lys Ile Lys Leu Gly Tyr Val
   565 570 575
   Ile Arg Ser Ile Asp Val Ala Asn Ser Gln Ile Leu His Thr Ser Arg
   580 585 590
   Gln Leu Ser Leu Asn Asn Tyr Leu Leu Ala Pro Asp Ser Phe Ala Val
   595 600 605
   Tyr Arg Ile Ile Asp Ser Gly Gly Ser Trp Phe Asp Ile Gly Ile Asp
   610 615 620
   Ser Asp Gly Phe Ser Phe Val Gly Val Ser Gln Ile Gly Lys Leu Glu
   625 630 635 640
   Phe Pro Leu Thr Ala Ser Tyr Met Gly Ile Gln Leu Ala Lys Ile Arg
   645 650 655
   Leu Ala Ser Asn Ile Arg Ser Gly Met Val Lys Ile
   660 665
<210> 8
   <211> 36
   <212> ADN
   <213> Séquence artificielle
<220>
   <223> Description de la séquence artificielle: oligonucleotide
<400> 8
   aaacgcgtcg acatgtgctc aacctgcgct aacgtg 36
<210> 9
   <211> 33
   <212> ADN
   <213> Séquence artificielle
<220>
   <223> Description de la séquence artificielle: oligonucleotide
<400> 9
   ttttgatatc tcatatttta accattccac tcc 33
<210> 10
   <211> 3701
   <212> ADN
   <213> virus canarypox
<400> 10
   aagcttctat caaaagtctt aatgagttag gtgtagatag tatagatatt actacaaagg 60
   tattcatatt tcctatcaat tctaaagtag atgatattaa taactcaaag atgatgatag 120
   tagataatag atacgctcat ataatgactg caaatttgga cggttcacat tttaatcatc 180
   acgcgttcat aagtttcaac tgcatagatc aaaatctcac taaaaagata gccgatgtat 240
   ttgagagaga ttggacatct aactacgcta aagaaattac agttataaat aatacataat 300
   ggattttgtt atcatcagtt atatttaaca taagtacaat aaaaagtatt aaataaaaat 360
   acttacttac gaaaaaatgt cattattaca aaaactatat tttacagaac aatctatagt 420
   agagtccttt aagagttata atttaaaaga taaccataat gtaatattta ccacatcaga 480
   tgatgatact gttgtagtaa taaatgaaga taatgtactg ttatctacaa gattattatc 540
   atttgataaa attctgtttt ttaactcctt taataacggt ttatcaaaat acgaaactat 600
   tagtgataca atattagata tagatactca taattattat atacctagtt cttcttcttt 660
   gttagatatt ctaaaaaaaa gagcgtgtga tttagaatta gaagatctaa attatgcgtt 720
   aataggagac aatagtaact tatattataa agatatgact tacatgaata attggttatt 780
   tactaaagga ttattagatt acaagtttgt attattgcgc gatgtagata aatgttacaa 840
   acagtataat aaaaagaata ctataataga tataatacat cgcgataaca gacagtataa 900
   catatgggtt aaaaatgtta tagaatactg ttctcctggc tatatattat ggttacatga 960
   tctaaaagcc gctgctgaag atgattggtt aagatacgat aaccgtataa acgaattatc 1020
   tgcggataaa ttatacactt tcgagttcat agttatatta gaaaataata taaaacattt 1080
   acgagtaggt acaataattg tacatccaaa caagataata gctaatggta catctaataa 1140
   tatacttact gattttctat cttacgtaga agaactaata tatcatcata attcatctat 1200
   aatattggcc ggatattttt tagaattctt tgagaccact attttatcag aatttatttc 1260
   ttcatcttct gaatgggtaa tgaatagtaa ctgtttagta cacctgaaaa cagggtatga 1320
   agctatactc tttgatgcta gtttattttt ccaactctct actaaaagca attatgtaaa 1380
   atattggaca aagaaaactt tgcagtataa gaactttttt aaagacggta aacagttagc 1440
   aaaatatata attaagaaag atagtcaggt gatagataga gtatgttatt tacacgcagc 1500
   tgtatataat cacgtaactt acttaatgga tacgtttaaa attcctggtt ttgattttaa 1560
   attctccgga atgatagata tactactgtt tggaatattg cataaggata atgagaatat 1620
   attttatccg aaacgtgttt ctgtaactaa tataatatca gaatctatct atgcagattt 1680
   ttactttata tcagatgtta ataaattcag taaaaagata gaatataaaa ctatgtttcc 1740
   tatactcgca gaaaactact atccaaaagg aaggccctat tttacacata catctaacga 1800
   agatcttctg tctatctgtt tatgcgaagt aacagtttgt aaagatataa aaaatccatt 1860
   attatattct aaaaaggata tatcagcaaa acgattcata ggtttattta catctgtcga 1920
   tataaatacg gctgttgagt taagaggata taaaataaga gtaataggat gtttagaatg 1980
   gcctgaaaag ataaaaatat ttaattctaa tcctacatac attagattat tactaacaga 2040
   aagacgttta gatattctac attcctatct gcttaaattt aatataacag aggatatagc 2100
   taccagagat ggagtcagaa ataatttacc tataatttct tttatcgtca gttattgtag 2160
   atcgtatact tataaattac taaattgcca tatgtacaat tcgtgtaaga taacaaagtg 2220
   taaatataat caggtaatat ataatcctat ataggagtat atataattga aaaagtaaaa 2280
   tataaatcat ataataatga aacgaaatat cagtaataga caggaactgg cagattcttc 2340
   ttctaatgaa gtaagtactg ctaaatctcc aaaattagat aaaaatgata cagcaaatac 2400
   agcttcattc aacgaattac cttttaattt tttcagacac accttattac aaactaacta 2460
   agtcagatga tgagaaagta aatataaatt taacttatgg gtataatata ataaagattc 2520
   atgatattaa taatttactt aacgatgtta atagacttat tccatcaacc ccttcaaacc 2580
   tttctggata ttataaaata ccagttaatg atattaaaat agattgttta agagatgtaa 2640
   ataattattt ggaggtaaag gatataaaat tagtctatct ttcacatgga aatgaattac 2700
   ctaatattaa taattatgat aggaattttt taggatttac agctgttata tgtatcaaca 2760
   atacaggcag atctatggtt atggtaaaac actgtaacgg gaagcagcat tctatggtaa 2820
   ctggcctatg tttaatagcc agatcatttt actctataaa cattttacca caaataatag 2880
   gatcctctag atatttaata ttatatctaa caacaacaaa aaaatttaac gatgtatggc 2940
   cagaagtatt ttctactaat aaagataaag atactctatc ttatctacaa gatatgaaag 3000
   aagataatca tttagtagta gctactaata tggaaagaaa tgtatacaaa aacgtggaag 3060
   cttttatatt aaatagcata ttactagaag atttaaaatc tagacttagt ataacaaaac 3120
   agttaaatgc caatatcgat tctatatttc atcataacag tagtacatta atcagtgata 3180
   tactgaaacg atctacagac tcaactatgc aaggaataag caatatgcca attatgtcta 3240
   atattttaac tttagaacta aaacgttcta ccaatactaa aaataggata cgtgataggc 3300
   tgttaaaagc tgcaataaat agtaaggatg tagaagaaat actttgttct ataccttcgg 3360
   aggaaagaac tttagaacaa cttaagttta atcaaacttg tatttatgaa cactataaaa 3420
   aaattatgga agatacaagt aaaagaatgg atgttgaatg tcgtagttta gaacataact 3480
   atacggctaa cttatataaa gtgtacggac aaaacgaata tatgattact tatatactag 3540
   ctctcataag taggattaat aatattatag aaactttaaa atataatctg gtggggctag 3600
   acgaatctac aatacgtaat ataaattata taatttcaca aagaacaaaa aaaaatcaag 3660
   tttctaatac cttatagata aactatattt tttaccactg a 3701
<210> 11
   <211> 42
   <212> ADN
   <213> Séquence artificielle
<220>
   <223> Description de la séquence artificielle: oligonucleotide
<400> 11
   atcatcgagc tcgcggccgc ctatcaaaag tcttaatgag tt 42
<210> 12
   <211> 73
   <212> ADN
   <213> Séquence artificielle
<220>
   <223> Description de la séquence artificielle: oligonucleotide
<400> 12
   gaattcctcg agctgcagcc cgggttttta tagctaatta gtcatttttt cgtaagtaag 60
   tatttttatt taa 73
<210> 13
   <211> 72
   <212> ADN
   <213> Séquence artificielle
<220>
   <223> Description de la séquence artificielle: oligonucleotide
<400> 13
   cccgggctgc agctcgagga attcttttta ttgattaact agtcaaatga gtatatataa 60
   ttgaaaaagt aa 72
<210> 14
   <211> 45
   <212> ADN
   <213> Séquence artificielle
<220>
   <223> Description de la séquence artificielle: oligonucleotide
<400> 14
   gatgatggta ccttcataaa tacaagtttg attaaactta agttg 45
<210> 15
   <211> 34
   <212> ADN
   <213> Séquence artificielle
<220>
   <223> Description de la séquence artificielle: oligonucleotide
<400> 15
   aaacccgggt tctttattct atacttaaaa agtg 34
<210> 16
   <211> 43
   <212> ADN
   <213> Séquence artificielle
<220>
   <223> Description de la séquence artificielle: oligonucleotide
<400> 16
   aaaagaattc gtcgactacg atacaaactt aacggatatc gcg 43
<210> 17
   <211> 55
   <212> ADN
   <213> Séquence artificielle
<220>
   <223> Description de la séquence artificielle: oligonucleotide
<400> 17
   aaatcgcgat atccgttaag tttgtatcgt aatgtgctca acctgcgcta acgtg 55
<210> 18
   <211> 33
   <212> ADN
   <213> Séquence artificielle
<220>
   <223> Description de la séquence artificielle: oligonucleotide
<400> 18
   ttttgtcgac tcatatttta accattccac tcc 33
<210> 19
   <211> 33
   <212> ADN
   <213> Séquence artificielle
<220>
   <223> Description de la séquence artificielle: oligonucleotide
<400> 19
   ttttgtcgac tcatagtttt gtcatagtac tcc 33
<210> 20
   <211> 33
   <212> ADN
   <213> Séquence artificielle
<220>
   <223> Description de la séquence artificielle: oligonucleotide
<400> 20
   tatgcggccg ccaccatgtg gctgcagaac ctg 33
<210> 21
   <211> 36
   <212> ADN
   <213> Séquence artificielle
<220>
   <223> Description de la séquence artificielle: oligonucleotide
<400> 21
   tatgcggccg ctacgtatca cttcttgact ggtttc 36
<210> 22
   <211> 432
   <212> ADN
   <213> Felis catus
<400> 22
   atgtggctgc agaacctgct tttcctgggc actgtggtct gcagcatctc tgcacccacc 60
   agttcaccca gctctgtcac tcggccctgg caacacgtgg atgccatcaa ggaggctctg 120
   agccttctga acaacagtag tgaaataact gctgtgatga atgaagcagt agaagtcgtc 180
   tctgaaatgt ttgaccctga ggagccgaaa tgcctgcaga ctcacctaaa gctgtacgag 240
   cagggcctac ggggcagcct catcagcctc aaggagcctc tgagaatgat ggccaaccat 300
   tacaagcagc actgcccctt tactccggaa acgccctgtg aaacccagac tatcaccttc 360
   aaaaatttca aagagaatct gaaggatttt ctgtttaaca tcccctttga ctgctggaaa 420
   ccagtcaaga ag 432
<210> 23
   <211> 432
   <212> ADN
   <213> Felis catus
<400> 23
   atgtggctgc agaacctgct tttcctgggc actgtggtct gcagcatctc tgcacccacc 60

## Description

La présente invention a trait à des gènes de souches particulières de calicivirus félins, aux protéines codées par ces gènes, et leur utilisation pour la réalisation de préparations immunogènes et de vaccins recombinés ou de sous-unités contre la calicivirose féline. Ces préparations immunogènes et ces vaccins peuvent également être combinés avec des préparations immunogènes ou des vaccins préparés sur la base d'autres agents pathogènes félins, pour la réalisation de préparations immunogènes et de vaccins multivalents.

Les calicivirus félins (en anglais Feline Calicivirus ou FCV) ont été décrits pour la première fois en 1957 (Fastier L.B. Am. J. Vet. Res. 1957. 18. 382-389). Les calicivirus félins sont avec les herpès virus félins les deux sources principales des affections virales du tractus respiratoire supérieur chez le chat. Les virus FCV touchent un grand nombre d'animaux, avec des taux de portage du FCV de l'ordre de 15 à 25 %, et une séroprévalence anti-FCV de 70 à 100 % (Coutts et al. Vet. Rec. 1994. 135. 555-556 ; Ellis T.M. Australian Vet. J. 1981. 57. 115-118 ; Harbour et al. Vet. Rec. 1991. 128. 77-80; Reubel et al. Feline Dendistry 1992. 22. 1347-1360). Après une phase initiale d'hyperthermie, ces affections respiratoires s'accompagnent généralement d'ulcérations buccales (palais, langue, lèvres, nez), de rhinite, de conjonctivite, éventuellement d'anorexie et d'asthénie. Les virus FCV peuvent également causer des pneumonies, des entérites, et des douleurs articulaires (syndrome de boiterie).

La transmission du virus FCV ne se fait qu'horizontalement, il n'y a pas de transmission verticale de la mère à son chaton lors de la gestation (Johnson R.P. Res. Vet. Sci. 1984. 31. 114-119). La transmission du FCV se fait par contact entre animaux infectés et animaux sains ou par voie aérienne lors d'éternuements (Wardley RC. Arch. Virol. 1976. 52. 243-249).

Les calicivirus félins sont des virus nus de la famille des *Caliciviridae,* ils sont pourvus d'un ARN positif simple brin, d'une taille d'environ 7,7 kilo paires de bases (kpb) (Carter M.J. Arch. Virol. 1994. 9. 429-439).

Comme de nombreux virus à ARN, une grande hétérogénéité existe au sein de la population virale de FCV. Les variations antigéniques, mises en évidence dès le début des années 70 par des expériences de séroneutralisations croisées, permettent de classer les FCV en plusieurs souches virales ou quasi-espèces (Radfoord et al. Proc. 1st Int. Symp. Caliciviruses ESVV 1997. 93-99).

Plusieurs souches de FCV ont été isolées et séquencées, notamment la souche F9 (Carter et al. Arch. Virol. 1992. 122. 223-235, séquence déposée dans la banque de données GenBank sous le numéro d'accès M86379), CFI (Neill et al. J. Virol. 1991. 65. 5440-5447, numéro d'accès GenBank U13992 et M32819), Urbana ou URB (Sosnovtsev et Green Virology 1995. 210. 383-390, numéro d'accès GenBank L40021), F4 (Tohya et al. Arch. Virol. 1991. 117. 173-181, numéros d'accès GenBank D31836 et D90357), KCD (Fastier L.B. Am. J. Vet. Res. 1957. 18. 882-889, numéro d'accès GenBank L09719), LLK (numéro d'accès GenBank U07131), NADC (numéro d'accès GenBank L09718), 2280 (numéro d'accès GenBank X99445) et 255 (Kahn et Gillepsie. Cornel Vet. 1970. 60. 669-683, numéro d'accès GenBank U07130).

La vaccination contre le FCV a été mise en place depuis la fin des années 70 à partir de souches FCV atténuées, principalement la souche F9 isolée en 1958 par Bittle (Bittle et al. Am. J. Vet. Res. 1960. 21. 547-550) ou des souches dérivées de F9 par passage *in vitro* ou *in vivo* (" F9-like ").

Des vaccins inactivés sont aussi disponibles. Ils utilisent les souches 255 et 2280, isolées respectivement en 1970 chez un chat présentant une pneumonie (Kahn et Gillepsie. Cornell Vet. 1970. 60. 669-683) et en 1983 chez un chat atteint de boiterie (Pedersen et al. Fel. Prac. 1983. 13. 26-35).

La réponse humorale est essentiellement dirigée contre la protéine de capside, aussi dénommée p65 (Guiver et al. J. Gen. Virol. 1992. 73. 2429-2433). Les gènes codant pour la protéine de capside de nombreux calicivirus félins ont été séquencés et comparés sans que l'on puisse distinguer plus particulièrement certaines séquences (Glenn et al. Proc. 1st Int. Symp. Caliciviruses ESVV 1997. 106-110 ; Geissler et al. Virus Res. 1997. 48. 193-206 ; Neill et al. Proc. 1st Int. Symp. Caliciviruses ESVV 1997. 120-124).

Le gène codant pour la protéine de capside a également été cloné et exprimé dans différents systèmes d'expression, notamment le gène codant pour la protéine de capside du virus FCV KS20 dans des plasmides (Geissler et al. J. Virol. 1999. 73. 834-838), les gènes codant les protéines de capside des virus FCV CFI-68, JOK63, JOK92, LS012 et F9 dans des baculovirus (DeSilver et al. Proc. 1st Int. Symp. Caliciviruses ESVV 1997. 131-143), le gène codant la protéine de capside du virus FCV F4 dans des virus herpès félins de type 1 (Yokoyama et al. J. Vet. Med. Sci. 1998. 60. 717-723). Ces différentes constructions ont permis la production de protéines de capside recombinées.

Du fait de la dérive antigénique dans le temps, les antisérums produits contre d'anciennes souches vaccinales isolées dans les années 60-70, telles que les souches F9, 255 ou 2280, ne neutralisent que peu d'isolats des années 90. Par exemple, le sérum anti-F9 neutralise 43 % des isolats de la période 1990-96, contre 56 % pour la période 1980-89 et 86 % pour la période 1958-79, et seulement 10 % des isolats anglais de la période 1990-96 (Lauritzen et al. Vet. Microbiol. 1997. 56. 55-63).

La présente invention a pour objectif la mise en évidence de souches de FCV, induisant chez les chats des anticorps ayant un large spectre de neutralisation croisée.

L'invention a en particulier pour objectif, à partir des souches sélectionnées, l'isolement et la caractérisation, de gènes codant pour des protéines immunogènes utilisables pour la vaccination contre la calicivirose féline.

Un autre objectif de l'invention est de proposer des vecteurs d'expression *in vitro* et *in vivo* recombinés contenant et exprimant au moins une telle séquence nucléotidique.

Un autre objectif encore de l'invention est de proposer des préparations immunogènes ou des vaccins contre la calicivirose féline.

Un autre objectif encore de l'invention est de proposer des préparations immunogènes multivalentes et des vaccins multivalents contre la calicivirose féline et contre au moins un autre pathogène félin.

L'invention concerne essentiellement deux souches FCV obtenues par écouvillonnages pharyngés pratiqués en France et au Royaume-Uni sur des chats présentant des signes d'infection par calicivirus félins. Il s'agit respectivement de la souche G1 (déposée à la Collection Nationale de Cultures de Microorganismes (ou CNCM) de l'Institut Pasteur, Paris, France, sous le numéro d'accès I-2167) et de la souche 431 (déposée à la CNCM sous le numéro d'accès I-2166), toutes les deux déposées le 12 mars 1999. Cette souche FCV G1 isolée en France ne correspond pas à la souche FCV isolée au Royaume-Uni en 1978 par Tohya (Tohya Y. et al. Jpn. J. Sci., 1990, 52, 955-961) et nommée également G1.

La sélection des souches FCV 431 et G1 a été réalisée par des tests de séroneutralisation croisée vis-à-vis des isolats FCV d'un panel de référence. Ce panel de référence est composé de 18 isolats actuels de FCV prélevés sur des chats présentant des signes d'infection par calicivirus félin et provenant de trois zones géographiques distinctes. 7 isolats sont américains, ces isolats sont identifiés RMI1, RMl2, RMl3, RMI5, RMI6, RMI7 et RMl9. 7 isolats sont français, ils sont désignés A2, F1, G1, G3, F3031, H3-2 et H1-4. Les 4 derniers isolats sont anglais, ils sont désignés 431, 388b, 337 et J5.

Les souches du panel sont accessibles auprès de la déposante sur simple demande. Elles ont aussi été publiées dans un article de la revue « Archives of Virology » (Poulet et al. Arch. Virol. février 2000. 145(2). 243-261), disponible en ligne sur Internet à la date de dépôt auprès de l'éditeur.

Lors de tests de séroneutralisations croisées entre les 18 isolats de FCV du panel de référence, il s'est avéré de manière surprenante que l'anti-sérum de l'isolat 431 neutralise 14 des 17 isolats hétérologues du panel de référence (le titre homologue de séroneutralisation n'est pas pris en compte). Par comparaison les anti-sérums des souches vaccinales " historiques " 255 et F9 ne neutralisent chacun que 2 des 18 isolats du panel.

De manière inattendue, la déposante a donc trouvé avec la souche FCV 431 une souche dominante qui peut être utilisée pour la protection des félidés et en particulier des chats contre la plupart des souches FCV. Grâce au panel de souches FCV divulguées ici, il est possible à l'homme du métier de sélectionner d'autres souches FCV dominantes. Par équivalence l'invention couvre aussi à travers la souche FCV 431 les souches FCV équivalentes à cette dernière, ayant des anticorps à large spectre de neutralisation croisée.

Il y a équivalence lorsque l'anti-sérum d'une souche FCV séroneutralise au moins 13 des 18 isolats hétérologues du panel de référence (c'est-à-dire incluant FCV 431), préférentiellement lorsqu'il séroneutralise au moins 14 des 18 isolats hétérologues du panel de référence, de manière encore plus préférée lorsqu'il séroneutralise au moins 15 des 18 isolats hétérologues du panel de référence.

On considère généralement qu'une souche de FCV séroneutralise une autre souche FCV lorsque le titre hétérologue de séroneutralisation est supérieur ou égal à 1,2 log₁₀ VN₅₀ (Povey C. et Ingersoll J., Infection and Immunity, 1975, 11, 877-885). La déposante a pris cette valeur comme seuil de positivité. Cependant, les résultats de séroneutralisation croisée obtenus par un isolat FCV ayant un titre homologue de séroneutralisation inférieur ou égal à 2 log₁₀ VN₅₀ ne peuvent être interprétés.

Une seconde méthode pour établir l'équivalence d'une souche FCV vis-à-vis de la souche FCV 431 est d'utiliser des anticorps monoclonaux spécifiques de la souche FCV 431 et de tester par immunofluorescence indirecte (IFI) la souche FCV candidate. La déposante a ainsi réussi à produire plusieurs anticorps monoclonaux qui se sont avérés spécifiques de la souche 431. L'un d'eux a été nommé 44. Il y a équivalence s'il y a une réactivité en immunofluorescence avec des anticorps monoclonaux spécifiques de 431, par exemple avec l'anticorps monoclonal 44. Cet anticorps monoclonal et l'hybridome correspondant sont disponibles auprès de la déposante sur simple demande et sont aussi divulgués dans l'article de Poulet *et al.* supra. L'hybridome correspondant a également été déposé le 11 août 1999 à la CNCM sous le numéro d'accès I-2282. Il va de soit cependant que l'homme du métier est parfaitement à même de produire des anticorps monoclonaux par les techniques classiques et de sélectionner, par rapport au panel, ceux qui sont spécifiques de la souche 431.

L'autre souche FCV G1 a été choisie pour sa complémentarité vis-à-vis de la souche FCV 431, à savoir que la combinaison des anti-sérums de 431 et de G1 séroneutralisent 100% des isolats du panel de référence, c'est-à-dire que la souche FCV G1 a un titre homologue de séroneutralisation supérieur ou égal à 2 log₁₀ VN₅₀ et des titres de séroneutralisation hétérologues supérieurs ou égaux à 1,2 log₁₀ VN₅₀ vis-à-vis des isolats FCV du panel de référence contre lesquels l'anti-sérum 431 ne séroneutralise pas ou faiblement (valeur inférieure à 1,2 log₁₀ VN₅₀). L'invention couvre également les souches FCV équivalentes ayant la même complémentarité vis-à-vis de la souche FCV 431. On peut aussi produire et sélectionner des anticorps spécifiques de cette souche, ce qui permet de déterminer des équivalents sur cette autre base.

La déposante a en outre réussi à isoler, caractériser et séquencer le gène de la capside de FCV 431 et FCV G1, la protéine de capside, et a déterminé les séquences d'ADNc (ADN complémentaire) correspondantes.

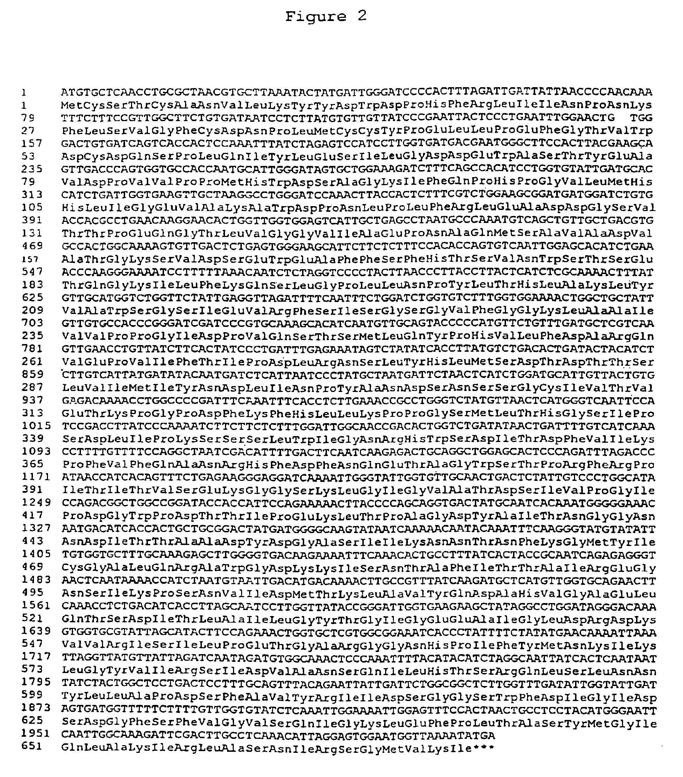

L'invention a donc pour objet un fragment d'acide nucléique comprenant tout ou partie de la séquence nucléotidique codant pour la protéine de capside du virus 431 dont la séquence en acides aminés est représentée à la SEQ ID NO: 7 ou à la figure 2, ou un fragment immunologiquement actif de cette protéine, c'est-à-dire un épitope, peptide ou polypeptide conservant substantiellement l'activité immunogène de la protéine de capside.

L'invention a notamment pour objet un fragment d'ADN comprenant la séquence d'ADNc de la SEQ ID NO : 6 ou un fragment conservant les propriétés essentielles de la séquence complète, c'est-à-dire codant pour un peptide, polypeptide ou épitope conservant substantiellement l'activité immunogène de la protéine de capside. L'invention a en particulier pour objet un fragment d'ADN comprenant cette séquence d'ADNc, notamment couplée avec des éléments de régulation de la transcription.

Il va de soi que l'invention recouvre automatiquement les fragments d'acide nucléique, fragments d'ADN et séquences d'ADNc équivalents, c'est-à-dire les fragments et séquences nucléotidiques spécifiques de capside FCV ne changeant pas la fonctionnalité ni la spécificité de souche de la séquence décrite ou des polypeptides codés par cette séquence. Seront bien entendu incluses les séquences qui diffèrent par dégénérescence du code.

L'invention recouvre également automatiquement les séquences nucléotidiques (ARN, ADN, ADNC) équivalentes à celles revendiquées. Il s'agit en particulier des séquences provenant de souches FCV équivalentes selon la définition donnée plus haut vis-à-vis du panel et/ou de l'anticorps monoclonal 44.

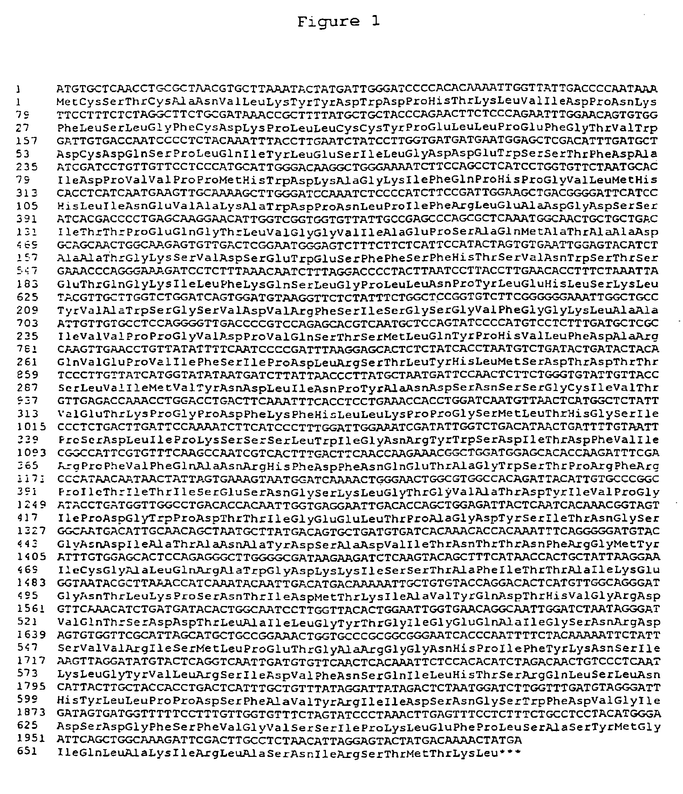

L'invention comprend un fragment d'acide nucléique comprenant tout ou partie de la séquence nucléotidique codant pour la protéine de capside du virus G1 telle que représentée à la SEQ ID NO : 5 ou à la figure 1, ou un fragment immunologiquement actif de cette protéine, c'est-à-dire un épitope, peptide ou polypeptide conservant substantiellement l'activité immunogène de la protéine de capside.

L'invention a notamment pour objet un fragment d'ADN comprenant la séquence d'ADNc de la SEQ ID NO : 4 ou un fragment conservant les propriétés essentielles de la séquence complète, c'est-à-dire codant pour un peptide, polypeptide ou épitope conservant substantiellement l'activité immunogène de la protéine de capside. L'invention a en particulier pour objet un fragment d'ADN comprenant cette séquence d'ADNc, notamment couplée avec des éléments de régulation de la transcription. Il va de soi que l'invention recouvre automatiquement les fragments d'acide nucléique, fragments d'ADN et séquences ADNc équivalents, c'est-à-dire les fragments et séquences nucléotidiques ne changeant pas la fonctionnalité ni la spécificité de souche de la séquence décrite ou des polypeptides codés par cette séquence. Seront bien entendu incluses les séquences qui diffèrent par dégénérescence du code.

L'invention recouvre également automatiquement les séquences nucléotidiques (ARN, ADN, ADNc) équivalentes à celles revendiquées. Il s'agit en particulier des séquences nucléotidiques provenant de souches FCV complémentaires selon le sens donné plus haut.

Le gène codant pour la protéine de capside des virus FCV G1 et FCV 431 a une taille de 2007 nucléotides pour FCV 431 et de 2010 nucléotides pour FCV G1. La protéine de capside a une taille de 668 acides aminés pour FCV 431 et de 669 acides aminés pour FCV G1, et une masse de 60-65 kDa (protéine p65).

L'invention a aussi pour objet un vecteur d'expression comprenant au moins un fragment d'ADN selon l'invention dans des conditions permettant son expression *in vivo*. Suivant une modalité particulière, le vecteur d'expression comprend un ADNc type 431 et un ADNc type G1. Par « type », il faut entendre que l'ADNc est complémentaire d'une séquence ARN de la souche considérée.

Ces vecteurs d'expression peuvent être des poxvirus, par exemple le virus de la vaccine, les avipox (canarypox, folwpox), y compris les poxvirus spécifiques d'espèce (swine pox, raccoonpox et camelpox), les adénovirus et les herpèsvirus, tels que les virus herpès félins (e.g. FR-A-2 741 806). Pour les Poxvirus, l'homme du métier peut se reporter à WO-A-9215672, WO-A-9526751, WO-A-9012882, et WO-A-9527780.

Plusieurs stratégies d'insertions peuvent être utilisées pour l'expression de plusieurs séquences nucléotidiques hétérologues à partir du même vecteur d'expression *in vivo.* Ces stratégies d'insertion sont notamment l'utilisation d'une double cassette d'expression ayant, une orientation opposée, ou l'utilisation d'une double cassette d'expression ayant une orientation identique, ou encore une cassette multiple d'expression avec un élément " IRES " (Internal Ribosome Entry Site) situé entre chaque insert (brevet EP-A1-0803573).

Les séquences nucléotidiques hétérologues sont insérées sous la dépendance de signaux régulateurs de la transcription et notamment de promoteurs, de préférence apportés lors de l'insertion. Il n'est toutefois pas exclu de faire exprimer ces séquences nucléotidiques hétérologues sous le contrôle de signaux propres au vecteur d'expression utilisé. Pour les vecteurs d'expression canarypox, un des promoteurs préférés est le promoteur vaccine H6 (Taylor J. et al. Vaccine, 1988, 6, 504-508 ; Guo P. et al. J. Virol., 1989, 63, 4189-4198 ; Perkus M. et al. J. Virol., 1989, 63, 3829-3836).

Les vecteurs d'expression *in vivo* peuvent également être des plasmides. Le terme plasmide entend recouvrir toute unité de transcription ADN sous forme d'une séquence polynucléotidique comprenant une séquence d'ADNc selon l'invention et les éléments nécessaires à son expression *in vivo.* On préféré la forme plasmide circulaire, superenroulée ou non. La forme linéaire entre également dans le cadre de cette invention.

Chaque plasmide comprend un promoteur apte à assurer, dans les cellules hôtes, l'expression de l'ADNc inséré sous sa dépendance. Il s'agit en général d'un promoteur eucaryote fort et en particulier d'un promoteur précoce du cytomégalovirus CMV-IE, d'origine humaine ou murine, ou encore éventuellement d'une autre origine telle que rat, cobaye. De manière plus générale, le promoteur est soit d'origine virale, soit d'origine cellulaire. Comme promoteur viral autre que CMV-IE, on peut citer le promoteur précoce ou tardif du virus SV40 ou le promoteur LTR du virus du Sarcome de Rous. Comme promoteur cellulaire, on peut citer le promoteur d'un gène du cytosquelette, tel que par exemple le promoteur de la desmine, ou encore le promoteur de l'actine. Un sous-fragment de ces promoteurs, conservant la même activité promotrice est compris dans la présente invention, e.g. les promoteurs CMV-IE tronqués selon WO-A-98/00166.

Lorsque plusieurs séquences hétérologues (ADNc et/ou gènes de FCV ou d'autres pathogènes félins) sont présents dans le même plasmide, ceux-ci peuvent être présentés dans la même unité de transcription ou dans deux unités différentes.

Les plasmides peuvent également comprendre d'autres éléments de régulation de transcription, tels que par exemple des séquences stabilisatrices du type intron, de préférence intron Il du gène de la β-globine de lapin (van Ooyen et al. Science, 1979, 206: 337-344), séquence signal de la protéine codée par le gène de l'activateur du plasminogène tissulaire (tPA ; Montgomery et al. Cell. Mol. Biol. 1997, 43: 285-292), et signal de polyadénylation (polyA), notamment du gène de l'hormone de croissance bovine (bGH) (US-A-5 122 458) ou du gène de la β-globine du lapin.

L'invention a également pour objet l'utilisation des ADNc selon l'invention pour la production *in vitro* des protéines de capside ou de leurs fragments et épitopes immunologiquement actifs et leur incorporation dans des préparations immunogènes et vaccins sous-unitaires.

L'invention a également pour objet une préparation immunogène ou un vaccin contre la calicivirose féline comprenant au moins un vecteur d'expression *in vivo* recombiné selon l'invention et un véhicule ou excipient acceptable sur le plan vétérinaire, et éventuellement un adjuvant.

La notion de préparation immunogène recouvre toute préparation susceptible, une fois administrée au chat, d'induire au moins une réponse immunitaire dirigée contre le pathogène félin considéré. Par vaccin, on entend une préparation capable d'induire une protection efficace.

De préférence, cette préparation immunologique ou ce vaccin comprend un vecteur d'expression *in vivo* dans lequel est inséré un ADNc type FCV 431, ce qui inclut ses équivalents.

Selon une première particularité très avantageuse, cette préparation immunologique ou ce vaccin comprend un vecteur d'expression dans lequel est inséré un ADNc type FCV 431, ce qui inclut ses équivalents, et un ADNc type FCV G1, ce qui inclut aussi les équivalents de ce dernier.

Selon une deuxième particularité très avantageuse, cette préparation immunologique ou ce vaccin comprend au moins deux vecteurs d'expression: dans le premier est inséré un ADNc type FCV 431, ce qui inclut ses équivalents, et dans le second un ADNc de la souche FCV G1, ce qui inclut aussi les équivalents de ce dernier.

Pour adjuver les préparations et vaccins conformes à l'invention, on peut utiliser tout adjuvant approprié connu de l'homme de l'art. Toutefois, on préfère soit les formuler sous forme d'émulsions huile-dans-l'eau, soit leur ajouter des polymères de l'acide acrylique ou méthacrylique ou des copolymères d'anhydride maléique et de dérivé alcényle, ou encore un lipide cationique contenant un sel d'ammonium quaternaire.

Parmi les polymères, on préfère les polymères de l'acide acrylique ou méthacrylique réticulés, notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 (incorporé par référence) décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'allylpentaérythritol. Parmi eux, on peut citer les Carbopol® 974P, 934P et 971 P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields et al., Nature, 186: 778-780, 4 juin 1960 (incorporé par référence). Sur le plan de leur structure, les polymères d'acide acrylique ou méthacrylique et les EMA® sont formés de préférence de motifs de base de formule suivante : 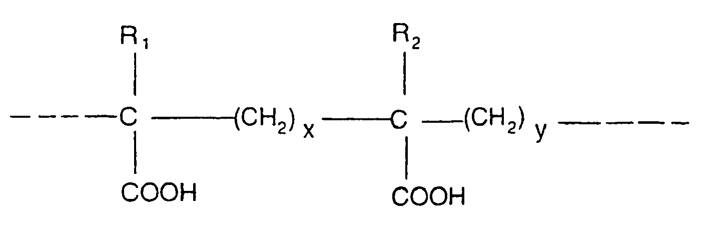 dans laquelle :
- R₁ et R₂, identiques ou différents, représentent H ou CH₃
- x = 0 ou 1, de préférence x = 1
- y=1 ou2,avecx+y=2

Pour les EMA®, x = 0 et y = 2. Pour les carbomères, x = y = 1.

Ces polymères sont dissous dans l'eau ou dans de l'eau physiologique (NaCl à 20 g/l) et le pH est ajusté à 7,3-7,4 par de la soude, pour donner la solution adjuvante dans laquelle le vecteur d'expression ou les sous-unités seront incorporés

La concentration en polymère dans la composition vaccinale finale sera de 0,01 % à 1,5 % P/V, plus particulièrement de 0,05 à 1 % P/V, de préférence de 0,1 à 0,4 % P/V.

Les lipides cationiques contenant un sel d'ammonium quaternaire, qui sont particulièrement mais pas exclusivement adaptés pour les vecteurs d'expression plasmidiques répondent à la formule : 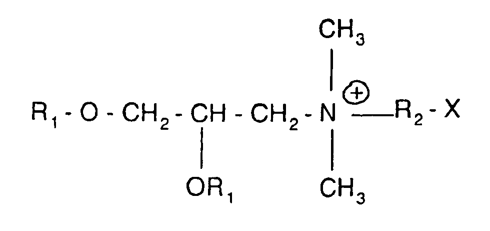 dans laquelle R1 est un radical aliphatique linéaire, saturé ou insaturé, ayant de 12 à 18 atomes de carbone, R2 est un autre radical aliphatique, renfermant 2 ou 3 atomes de carbone, et X un groupement hydroxyle ou amine.

On préfère le DMRIE (N-(2-hydroxyéthyl)-N,N-diméthyl-2,3-bis(tetradécyloxy)-1-propanammonium ; WO-A-9634109), de préférence couplé avec un lipide neutre, notamment le DOPE (dioléoyl-phosphatidyl-éthanolamine), pour former le DMRIE-DOPE. De préférence, le mélange plasmide avec cet adjuvant se fait de manière extemporanée et l'on préfère, avant son administration à l'animal, laisser le temps au mélange ainsi constitué de se complexer, par exemple pendant une durée allant de 10 à 60 minutes, notamment de l'ordre de 30 minutes.

Lorsque du DOPE est présent, le ratio molaire DMRIE : DOPE va de préférence de 95 : 5 à 5 : 95, plus particulièrement de 1 : 1.

Le ratio pondéral plasmide : adjuvant DMRIE ou DMRIE-DOPE peut aller notamment de 50 : 1 à 1 : 10, notamment de 10 : 1 à 1 : 5, de préférence de 1 : 1 à 1 : 2.

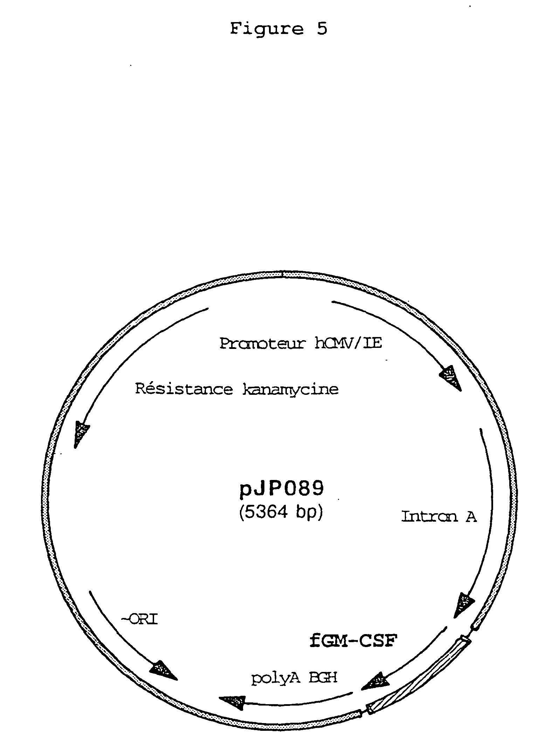

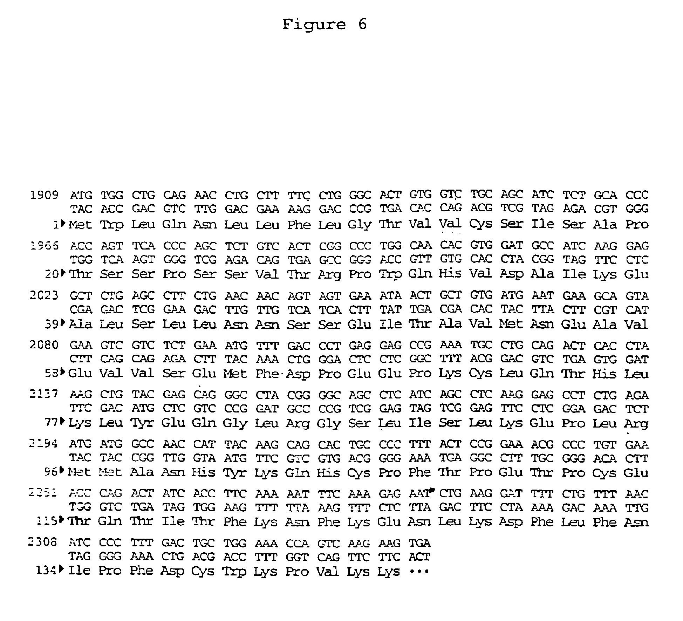

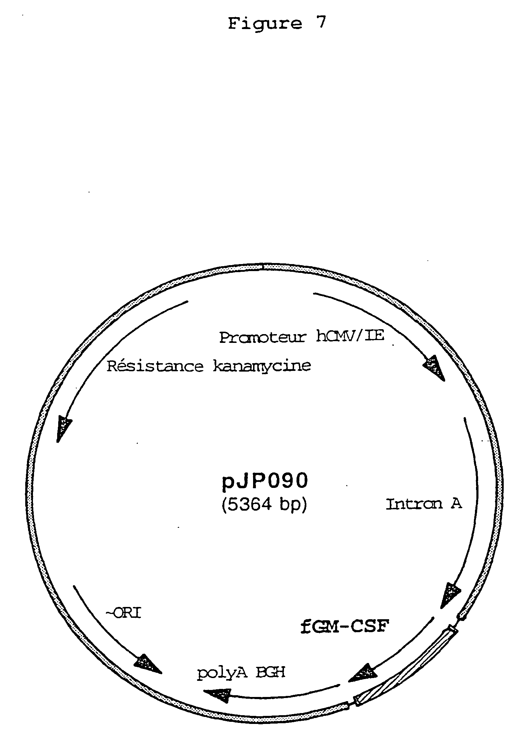

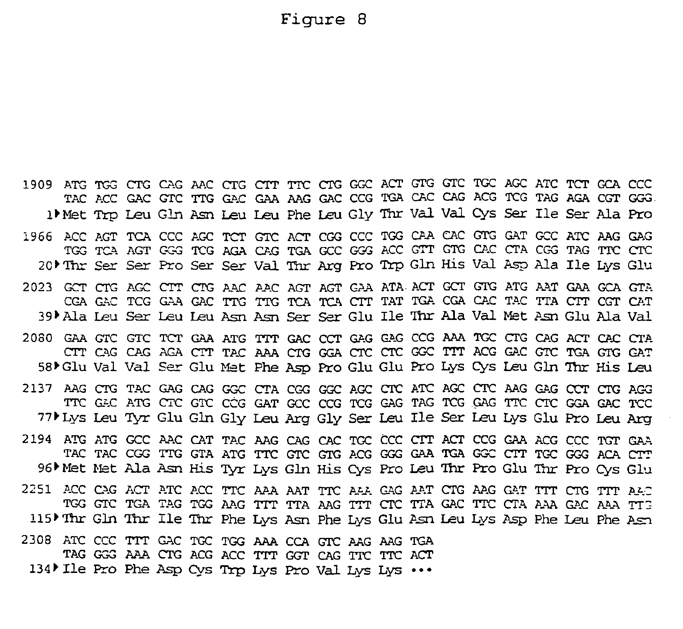

Les vecteurs d'expression *in vivo* codant pour un ADNc type FCV selon l'invention peuvent également coder pour le GM-CSF félin ou être associés à un second vecteur codant pour le GM-CSF félin (e.g. les plasmides pJP089 et pJP090, respectivement figure 5 et figure 6). Dans le cas de plasmides, on préfère un mélange de deux plasmides. Dans le cas de vecteurs viraux, on préfère un seul vecteur. Ce vecteur d'expression ou ce mélange de vecteurs d'expression peut aussi être adjuvé tel que décrit précédemment.

L'invention a aussi pour objet une préparation immunogène multivalente ou un vaccin multivalent contre la calicivirose féline et contre au moins un autre pathogène félin, utilisant un même vecteur d'expression *in vivo* recombiné contenant et exprimant au moins un ADNc type FCV selon l'invention et au moins une séquence nucléotidique d'un immunogène d'un autre pathogène félin ou d'un fragment immunologiquement actif de cet immunogène.

L'invention a également pour objet une préparation immunogène multivalente ou un vaccin multivalent comprenant au moins un vecteur d'expression *in vivo* dans lequel est insérée au moins un ADNc type FCV selon l'invention et au moins un deuxième vecteur d'expression dans lequel est insérée une séquence codant pour un immunogène, ou un fragment immunologiquement actif, d'un autre pathogène félin. Des plasmides appropriés dans lesquels est insérée une séquence codant pour un immunogène, ou un fragment immunologiquement actif, d'un autre pathogène félin, peuvent être notamment ceux décrits dans les exemples 7 à 15 et 17 à 19 de la demande de brevet WO-A-9803660 (pPB179, pPB180, pPB181, pAB009, pAB053, pAB052, pAB056, pAB058, pAB029, pAB030, pAB083, pAB041).

Les vaccins recombinés monovalents ou multivalents tels que décrits précédemment peuvent également être associés avec au moins un vaccin classique (inactivé, vivant atténué, sous-unités) dirigé contre au moins un pathogène félin identique ou différent.

Lesdits autres pathogènes félins sont notamment choisis parmi le groupe comprenant le virus de la rhinotrachéite féline ou virus herpès félin (FHV), le virus de la leucémie féline (FeLV), les parvovirus félins (FPV), le virus de la péritonite infectieuse féline (FIPV), le virus de l'immunodéficience féline (FIV), le virus de la rage, *Chlamydia.*

L'invention comprend les protéines de capside isolées, purifiées ou synthétiques, de la souche FCV431 et de la souche FCV G1, ayant la séquence en acides aminés représentée à la SEQ ID NO: 7, respectivement SEQ ID . NO : 5. Cela recouvre automatiquement les protéines équivalentes, c'est-à-dire issues de souches équivalentes aux souches FCV 431 et FCV G1 suivant les définitions données plus haut (mise en oeuvre du panel et/ou d'un anticorps monoclonal, notamment l'anticorps monoclonal 44). Avantageusement ces protéines de capside peuvent être assemblées sous forme de capsides vides.

L'invention a aussi pour objet les fragments et épitopes (au moins environ 8 à 10 acides aminés) de ces protéines, qui conservent la spécificité et l'immunogénicité de la protéine entière.

Les protéines de capside, éventuellement assemblées sous forme de capsides vides, et leurs fragments et épitopes, peuvent être produits par expression *in vitro.* La séquence nucléotidique correspondante est insérée dans un système d'expression *in vitro* et exprimée par ce système, et le produit d'expression récolté et éventuellement purifié, comme cela est connu en soi.

Le système d'expression peut être d'origine virale, en particulier le baculovirus (US-A-4 745 051). On intégre la séquence codante ou un fragment (dans le cas de l'épitope ou du fragment) dans le génome du baculovirus (e.g. le baculovirus Autographa californica Nuclear Polyhedrosis Virus AcNPV) et ce dernier est ensuite propagé, notamment sur cellules d'insectes, e.g. Spodoptera frugiperda Sf9 (dépôt ATCC CRL 1711).

Le système d'expression *in vitro* peut être d'origine procaryote, e.g. *Escherichia coli,* ou eucaryote, en particulier levures, e.g. *Saccharomyces cerevisiae,* ou des cellules eucaryotes de mammifères, notamment des lignées cellulaires telles que CHO (cellules ovariennes de hamster), HeLa, BHK ou des cellules d'insectes, e.g. Spodoptera frugiperda (supra), ou encore des cellules félines.

Comme promoteurs utilisables dans ces constructions cellulaires, on peut citer les promoteurs viraux forts tels que ceux du virus SV40 (Fiers et al., Nature, (1978) 273 : 113) et le promoteur précoce (CMV-IE) du virus CMV ou cytomegalovirus humain (McGregor et Caskey, Nucleic Acids Res. 17 : 2365, 1989), murin ou d'une autre origine, ou encore celui du gène polyhédrine du Baculovirus AcNPV (Hooft van Iddekinge et al., 1983, Virology 131 : 561-565).

L'homme de l'art sait comment purifier et/ou isoler les protéines, assemblées ou non sous forme de capsides vides, leurs fragments et épitopes à partir du produit des techniques décrites ci-dessus. A titre d'exemple, on peut rappeler que l'homme de l'art a à sa disposition différentes méthodes qui comprennent notamment : précipitation basée sur la solubilité des protéines, fragments et épitopes d'intérêt en fonction des conditions salines du milieu, précipitation par des solvants organiques, des polymères et autres matières, précipitation d'affinité et dénaturation sélective, chromatographie sur colonne, y compris chromatographie liquide haute performance (HPLC), chromatographie d'échange d'ions, chromatographie d'affinité, chromatographie d'immunoaffinité, chromatographie utilisant des ligands, immunoprécipitation, filtration sur gel, électrophorèse, méthodes de filtration, notamment ultrafiltration, et ultra-centrifugation sur gradient.

L'homme de l'art peut se reporter à titre d'exemple à K.Y. Green et al., J. Clin. Microb., Juillet 1997, Vol 35, 7 : 1909-1914, pour la production de capsides en baculovirus propagé sur cellules Sf9 et récolte par ultra-centrifugation sur des gradients de saccharose (10 à 50 %).

Les protéines de capside, et leurs fragments et épitopes, peuvent aussi être produits par synthèse chimique par les méthodes à la disposition de l'homme de l'art.

L'invention comprend les préparations immunogènes et vaccins comprenant au moins un antigène sous-unitaire formé d'une protéine de capside, de préférence assemblée sous forme de capsides vides, de FCV 431 et/ou FCV G1, ou d'un fragment ou épitope correspondant, dans un véhicule ou excipient acceptable au plan vétérinaire, et de préférence un adjuvant. De préférence, les préparations et vaccins selon l'invention comprennent des antigènes sous-unitaires issus des deux souches FCV 431 et FCV G1. De même, les préparations et vaccins peuvent comprendre des protéines de capside non assemblées et des protéines assemblées sous forme de capsides vides.

Pour adjuver les préparations immunogènes et vaccins sous-unitaires selon l'invention, on peut utiliser à titre d'adjuvant (1) de l'hydroxyde d'alumine, (2) un polymère de l'acide acrylique ou méthacrylique, un polymère d'anhydride maléique et de dérivé alcényle (décrits ci-dessus), ou (3) formuler la préparation immunogène ou vaccin sous forme d'une émulsion huile-dans-l'eau en particulier l'émulsion SPT décrite p 147 « Vaccine Design, The Subunit and Adjuvant Approach » edited by M. Powell, M. Newman, Plenum Press 1995, et l'émulsion MF59 décrite p183 du même ouvrage.

L'émulsion huile-dans-l'eau peut notamment être à base d'huile de paraffine liquide légère (type Pharmacopée européenne) ; d'huile isoprénoide telle que le squalane, le squalène; d'huile résultant de l'oligomérisation d'alcènes, en particulier d'isobutène ou de decène ; des esters d'acides ou d'alcools à groupement alkyle linéaire, plus particulièrement les huiles végétales, l'oléate d'éthyle, le di(caprylate / caprate) de propylène glycol, le tri(caprylate /caprate) de glycérol, le dioléate de propylène glycol ; des esters d'acides ou d'alcools gras ramifiés, en particulier des esters de l'acide isostéarique. L'huile est utilisée en association avec des émulsifiants pour former l'émulsion. Les émulsifiants sont de préférence des tensio-actifs non ioniques en particulier les esters de sorbitan, de mannide, de glycérol, de polyglycérol, de propylène glycol et de l'acide oléique, isostéarique, ricinoléique, hydroxystéarique, éventuellement éthoxylés, les blocs copolymères polyoxypropylène-polyoxyéthylène en particulier les Pluronic®, notamment L121.

L'invention a aussi pour objet les préparations immunogènes et vaccins multivalents dans lesquels la valence FCV est une valence sous-unitaire comme décrit ci-dessus.

La présente invention a aussi pour objet une méthode d'immunisation des chats, contre les maladies dues aux virus de la calicivirose féline.

Cette méthode comprend l'administration d'une préparation immunologique ou d'un vaccin selon l'invention à des chats. Cette administration peut se faire notamment par la voie parentérale, par administration sous-cutanée, intradermique, intramusculaire, intrapéritonéale. De préférence, l'administration se fait par la voie sous-cutanée ou intramusculaire.

Différents moyens d'administration peuvent être utilisés pour les préparations immunogènes et les vaccins plasmidiques, notamment des particules d'or enrobées d'ADN et projetées de façon à pénétrer dans les cellules de la peau du sujet à immuniser (Tang et al. Nature 1992. 356. 152-154) et les injecteurs par jet liquide permettant de transfecter à la fois des cellules de la peau et des cellules des tissus sous-jacents (Furth et al. Analytical Bioch. 1992. 205. 365-368).

L'homme de l'art possède les compétences nécessaires pour définir précisément le nombre d'administration et les doses à utiliser pour chaque protocole d'immunisation.

L'invention va être maintenant décrite plus en détail à l'aide de modes de réalisation pris à titre d'exemples non limitatifs et se référant au dessin dans lequel :
- **Figure 1** :: Séquence d'ADNc et de la protéine « capside » de la souche FCV G1
- **Figure 2** :: Séquence d'ADNc et de la protéine « capside » de la souche FCV 431
- **Figure 3** :: Carte de restriction du plasmide pJCA151
- **Figure 4** :: Séquence de la région C6L du génome du canarypox virus (souche ALVAC)
- **Figure 5 :**: Carte de restriction du plasmide pJP089
- **Figure 6** :: Séquence du gène GM-CSF félin 3R3
- **Figure 7** :: Carte de restriction du plasmide pJP090
- **Figure 8 :**: Séquence du gène GM-CSF félin 3R4
- **Figure 9** :: Tableau des titres de séroneutralisation croisée

### Liste des séquences pour les constructions de la présente invention

**SEQ ID NO 1 :** Oligonucléotide PB331
**SEQ ID NO 2** : Oligonucléotide PB333
**SEQ ID NO 3 :** Oligonucléotide PB332
**SEQ ID NO 4** : Séquence d'ADNc capside FCV G1
**SEQ ID NO 5** : Séquence de la protéine capside FCV G1
**SEQ ID NO 6** : Séquence d'ADNc capside FCV 431
**SEQ ID NO 7** : Séquence de la protéine capside FCV 431
**SEQ ID NO 8** : Oligonucléotide JCA289
**SEQ ID NO 9 :** Oligonucléotide JCA290
**SEQ ID NO 10 :** Séquence de la région C6L du canarypox virus (souche ALVAC)
**SEQ ID NO 11 :** Oligonucléotide C6A1
**SEQ ID NO 12 :** Oligonucléotide C6B1
**SEQ ID NO 13 :** Oligonucléotide C6C1
**SEQ ID NO 14 :** Oligonucléotide C6D1
**SEQ ID NO 15 :** Oligonucléotide JCA291
**SEQ ID NO 16 :** Oligonucléotide JCA292
**SEQ ID NO 17 :** Oligonucléotide JCA293
**SEQ ID NO 18 :** Oligonucléotide JCA294
**SEQ ID NO 19 :** Oligonucléotide JCA295
**SEQ ID NO 20 :** Oligonucléotide JP578
**SEQ ID NO 21 :** Oligonucléotide JP579
**SEQ ID NO 22 :** Séquence du gène GM-CSF félin 3R3
**SEQ ID NO 23:** Séquence du gène GM-CSF félin 3R4

### EXEMPLES

Toutes les constructions de plasmides ont été réalisées en utilisant les techniques standards de biologie moléculaire (clonage, digestion par les enzymes de restriction, synthèse d'un ADN complémentaire simple brin, amplification en chaîne par polymérase, élongation d'un oligonucléotide par une ADN polymérase...) décrites par Sambrook J. et al. (Molecular Cloning: A Laboratory Manual. 2nd Edition. Cold Spring Harbor Laboratory Press. Cold Spring Harbor. New York. 1989). Tous les fragments de restriction utilisés pour la présente invention, ainsi que les divers fragments d'amplification en chaîne par polymérase (= ACP ou PCR), ont été isolés et purifiés en utilisant le kit "Geneclean7" (BIO101 Inc. La Jolla, CA).

### Exemple 1 : Isolement et culture souches de calicivirus félin G1 et 431

La souche de calicivirus félin désignée G1 a été obtenue à partir d'un prélèvement réalisé en France sur un chat présentant des signes de calicivirose. Cette souche FCV G1 a été déposée le 12 mars 1999 à la Collection Nationale de Cultures de Microorganismes (ou CNCM) de l'Institut Pasteur, Paris, France, sous le numéro d'accès I-2167.

La souche de calicivirus félin désignée 431 a été isolée à partir d'un prélèvement réalisé en Angleterre, sur un chat présentant des signes de calicivirose. Cette souche FCV 431 a été déposée le 12 mars 1999 à la Collection Nationale de Cultures de Microorganismes (ou CNCM) de l'Institut Pasteur, Paris, France, sous le numéro d'accès I-2166.

Pour leur amplification, les souches de calicivirus félin ont été cultivées sur cellules de la lignée de rein de chat (Crandell-Reese Feline Kidney ou CRFK, N° ATCC CCL-94, Crandell et al. In Vitro 1973, 9, 176-185).

Les cellules CRFK sont mises en culture en plaque de 96 puits ou en Falcon 25 cm² avec du milieu DMEM supplémenté de 5 % de sérum de veau foetal contenant environ 100 000 cellules par ml. Les cellules sont cultivées à +37°C en atmosphère contenant 5% de CO₂.

Après 3 jours la couche cellulaire arrive à confluence. Le milieu de culture est alors remplacé par du milieu DMEM sans sérum mais additionné de 50 mg/l de gentamycine et les isolats viraux FCV sont ajoutées à raison d'un volume de 100 µl de dilutions sériées de quart en quart par puit pour le clonage en dilutions limites des virus FCV ou de 1 ml par Falcon.

Lorsque l'effet cytopathique (CPE) est complet (24-48 heures après le début de la mise en culture), les suspensions virales sont récoltées et congelées à -70°C. 3 à 4 passages successifs sont généralement nécessaires à la production d'un lot viral. Le lot viral est stocké à -70°C.

### Exemple 2 : Extraction de l'ARN viral des souches G1 et 431 de calicivirus félin

Les cellules CRFK sont cultivées à 37°C en flacons roulants de 2 litres (850 cm²) en milieu Eagle modifié (MEM, Gibco BRL) supplémenté avec 2,5 % d'hydrolysat de lactalbumine (Gibco BRL) et de 5% de sérum de veau foetal (Gibco BRL). On ajoute par flacon roulant 300 ml d'une suspension cellulaire en milieu MEM à environ 100 000 cellules/ml. Après 3 jours la couche cellulaire est confluente. Le milieu de culture cellulaire est alors remplacé par du milieu MEM sans sérum et le virus FCV ajouté à une multiplicité d'infection (moi) de 0,5 DICC₅₀/cellule. La culture virale est maintenue à 37°C pendant 24 à 48 heures jusqu'à l'obtention d'un effet cytopathique pour l'ensemble du tapis cellulaire. La suspension virale est récoltée puis clarifiée par centrifugation.

L'ARN viral contenu dans 100 ml de suspension virale de la souche FCV G1, qui vient d'être préparée, a été extrait avec les solutions du kit « High Pure^{™} Viral RNA Kit » Cat # 1 858 882, Roche Molecular Biochemicals), en suivant les instructions du fournisseur pour les étapes d'extraction. Le culot d'ARN obtenu à la fin de l'extraction a été re-suspendu avec 10 ml d'eau distillée stérile sans RNase.

L'ARN viral de la souche FCV 431 a été extrait dans les mêmes conditions à partir de 100 ml de suspension virale de la souche correspondante. Le culot d'ARN obtenu à la fin de l'extraction a été re-suspendu avec 10 ml d'eau distillée stérile sans RNase.

### Exemple 3 : Synthèse des ADN complémentaires des gènes capsides des souches G1 et 431 de calicivirus félin

Les ADN complémentaires correspondant aux gènes capsides des souches G1 et 431 de calicivirus félin ont été synthétisés avec le kit « Gene Amp RNA PCR Kit » (Cat # N 808 0017, Perkin-Elmer, Norwalk, CT 06859, USA) en utilisant les conditions données par le fournisseur.

Pour la souche FCV G1, une réaction de transcription inverse, suivie d'une réaction d'amplification en chaîne (Réaction « TI-ACP » ou « RT-PCR ») a été réalisée avec 1 ml de la suspension d'ARN viral FCV G1 (exemple 2) et avec les oligonucléotides suivants :
PB331 (33 mer) (SEQ ID NO 1)
   5' TTGCGGCCGCTGTGATGTGTTCGAAGTTTGAGC 3'
et PB333 (36 mer) (SEQ ID NO 2)
   5' TTGGCGCCGYTGACCMAGTGCAGCYTTRTCCAATTC 3'

Les conditions de synthèse du premier brin d'ADN complémentaire sont une température de 42°C pendant 15 min, puis 99°C pendant 5 min, et enfin 4°C pendant 5 min. Les conditions de la réaction ACP sont une température de 95°C pendant 2 min, puis 35 cycles (95°C pendant 1 min, puis 62°C pendant 1 min, et 72°C pendant 2 min), et enfin 72°C pendant 7 min pour produire un fragment RT-PCR d'environ 2000 paires de bases (pb) qui a été identifié « G1-4 ».

Pour la souche FCV 431, une réaction de transcription inverse, suivie d'une réaction d'amplification en chaîne (Réaction « TI-ACP » ou « RT-PCR ») a été réalisée avec 1 ml de la suspension d'ARN viral FCV 431 (exemple 2) et avec les oligonucléotides suivants :
PB331 ( 33 mer) (SEQ ID NO 1)
et PB332 (38 mer) (SEQ ID NO 3)
   5' TTGGCGCCAAYWGTRTTWGHTACAGTRTCAATYARRCC 3'

Les conditions de synthèse du premier brin d'ADN complémentaire sont une température de 42°C pendant 15 min, puis 99°C pendant 5 min, et enfin 4°C pendant 5 min. Les conditions de la réaction ACP sont une température de 95°C pendant 2 min, puis 35 cycles (95°C pendant 1 min, puis 62°C pendant 1 min, et 72°C pendant 2 min), et enfin 72°C pendant 7 min pour produire un fragment RT-PCR d'environ 2000 paires de bases (pb) qui a été identifié « 431-2 ».

### Exemple 4 : Clonage du gène codant pour la protéine capside de la souche G1 du calicivirus félin

Le fragment RT-PCR « G1-4 » a été digéré par Narl puis par Notl pour isoler, après électrophorèse en gel d'agarose, le fragment Narl-Notl d'environ 2000 pb. Ce fragment a été ligaturé avec le vecteur pBlueScript® II KS+ (Cat # 212208 Stratagene Inc., La Jolla, CA 92037, USA), préalablement digéré par Notl et Clal, puis déphosphorylé, pour donner le plasmide pG1-4-5 (5033 pb).

Le fragment Notl-Narl cloné dans ce plasmide a été entièrement séquencé sur les deux brins pour déterminer la séquence du gène capside. La séquence du gène capside de la souche FCV G1 (2010 pb) (SEQ ID NO 4), ainsi que la séquence de la protéine capside (p65) (668 acides aminés) (SEQ ID NO 5) codée par ce gène, sont présentées sur la figure 1.

### Exemple 5 : Clonage du gène codant pour la protéine capside de la souche 431 du calicivirus félin

Le fragment RT-PCR « 431-2 » a été digéré par Narl puis par Notl pour isoler, après électrophorèse en gel d'agarose, le fragment Narl-Notl d'environ 2000 pb. Ce fragment a été ligaturé avec le vecteur pBlueScript® Il KS+ (Cat # 212208 Stratagene Inc., La Jolla, CA 92037, USA), préalablement digéré par Notl et Clal, puis déphosphorylé, pour donner le plasmide p431-2-1 (4985 pb).

Le fragment Notl-Narl cloné dans ce plasmide a été entièrement séquencé sur les deux brins pour déterminer la séquence du gène capside. La séquence du gène capside de la souche FCV 431 (2007 pb) (SEQ ID NO 6), ainsi que la séquence de la protéine capside (p65) (668 acides aminés) (SEQ ID NO 7) codée par ce gène, sont présentées sur la figure 2.

### Exemple 6 : Construction du plasmide pJCA151

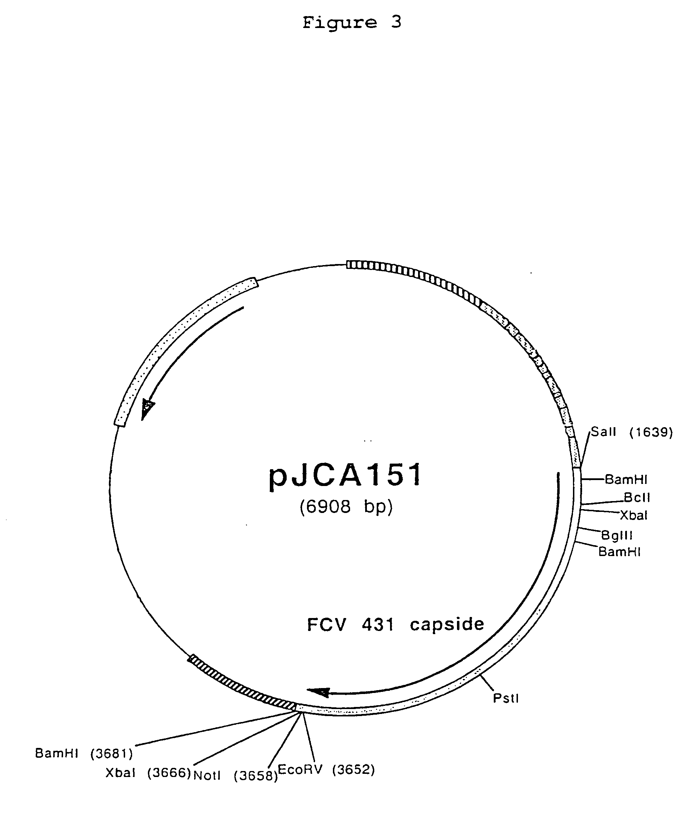

Une réaction ACP a été réalisée en utilisant le plasmide p431-2-1 (exemple 5) comme matrice et les oligonucléotides suivants :
JCA289 SEQ ID NO 8 (36 mer) :
   5' AAACGCGTCGACATGTGCTCAACCTGCGCTAACGTG 3'
et JCA290 SEQ ID NO 9 (33 mer) :
   5' TTTTGATATCTCATATTTTAACCATTCCACTCC 3'
pour amplifier un fragment ACP d'environ 2030 pb. Ce fragment a été digéré avec les enzymes de restriction Sall et EcoRV pour isoler, après électrophorèse en gel d'agarose, un fragment Sall-EcoRV de 2014 pb. Ce fragment de restriction a alors été ligaturé avec le plasmide pVR1012 (Hartikka J. et al. Human Gene Therapy. 1997. 7. 1205-1217), préalablement digéré par Sali et EcoRV, pour donner le plasmide pJCA151 (6908 pb) (Figure 3).

### Exemple 7 : Construction du plasmide donneur pour l'insertion dans le site C6L du canarypox virus

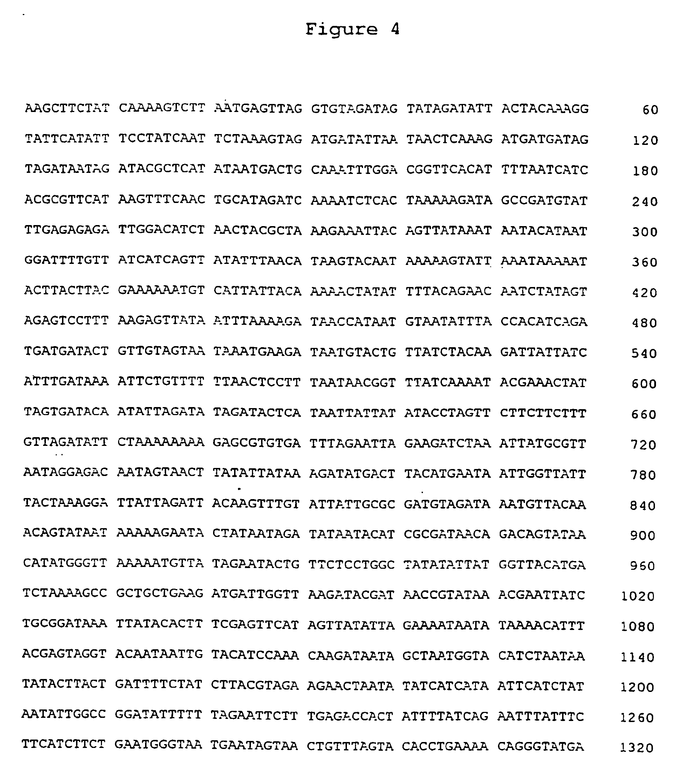

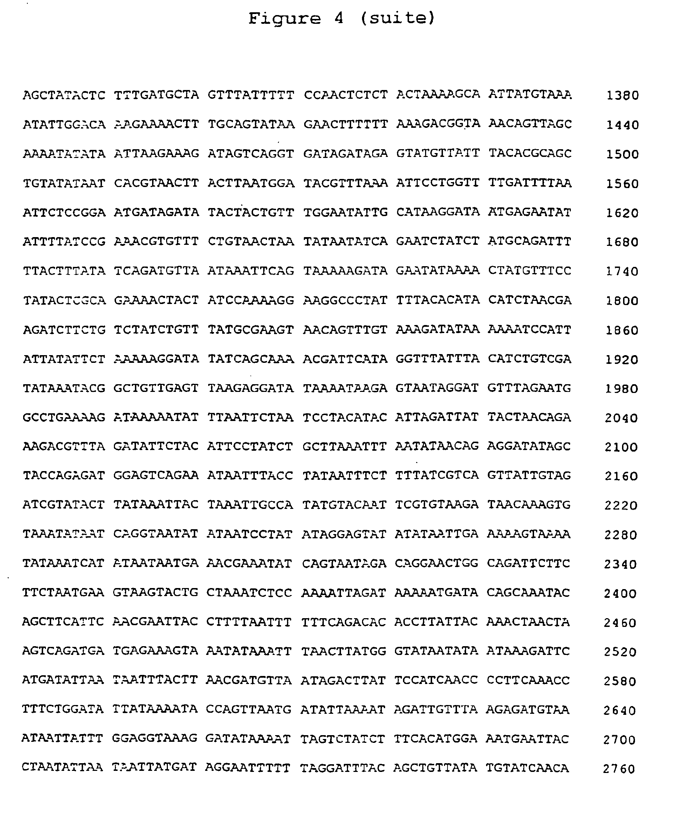

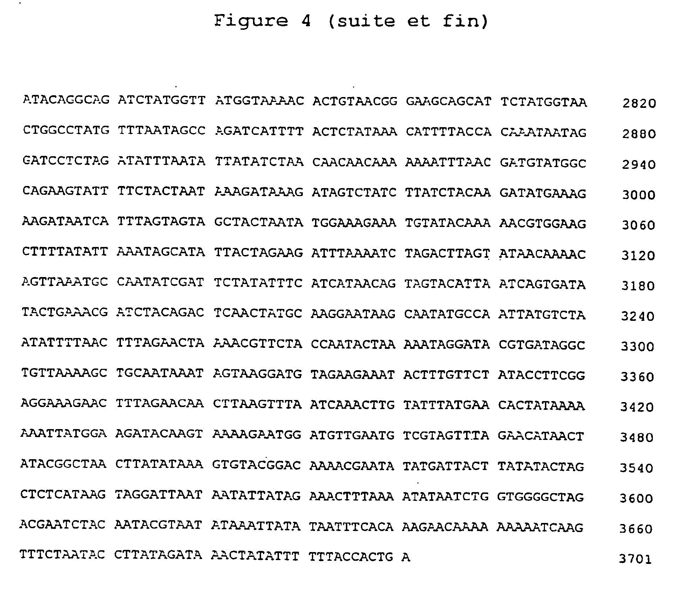

La figure 4 (SEQ ID NO 10) montre la séquence d'un fragment de 3700 pb de l'ADN génomique du canarypox virus. L'analyse de cette séquence a révélé un cadre ouvert de lecture (COL) qui a été appelé C6L, qui commence à la position 377 et se termine à la position 2254. La construction d'un plasmide d'insertion aboutissant à la délétion du COL C6L et à son remplacement par un site de clonage multiple flanqué de signaux d'arrêt de transcription et de traduction a été réalisée comme décrit ci-après.

Une réaction ACP a été réalisée à partir de la matrice constituée par l'ADN génomique du canarypox virus et avec les oligonucléotides suivants :
C6A1 (SEQ ID NO 11) (42 mer)
   5' ATCATCGAGCTCGCGGCCGCCTATCAAAAGTCTTAATGAGTT 3'
et C6B1 (SEQ ID NO 12) (73 mer)
pour isoler un fragment ACP de 432 pb (fragment A).

Une réaction ACP a été réalisée à partir de la matrice constituée par l'ADN génomique du canarypox virus et avec les oligonucléotides suivants:
C6C1 (SEQ ID NO 13) (72 mer)
et C6D1 (SEQ ID NO 14) (45 mer)
   5' GATGATGGTACCTTCATAAATACAAGTTTGATTAAACTTAAGTTG 3'
pour isoler un fragment ACP de 1210 pb (fragment B).

Les fragments A et B ont été hybridés ensemble pour servir de matrice à une réaction ACP réalisée avec les oligonucléotides C6A1 (SEQ ID NO 11) et C6D1 (SEQ ID NO 14) pour générer un fragment ACP de 1630 pb. Ce fragment a été digéré par les enzymes de restriction Sacl et Kpnl, pour isoler, après électrophorèse en gel d'agarose, un fragment Sacl-Kpnl de 1613 pb. Ce fragment a été ligaturé avec le vecteur pBlueScript® II SK+ (Stratagene, La Jolla, CA, USA, Cat # 212205), préalablement digéré par les enzymes Sacl et Kpnl , pour donner le plasmide pC6L. La séquence de ce plasmide a été vérifiée par séquençage. Ce plasmide contient 370 pb de séquences situées en amont du COL C6L (« bras flanquant gauche C6 »), un signal vaccine d'arrêt précoce de transcription, des codons stops dans les 6 phases de lecture, un site de clonage multiple contenant les sites de restriction Smal, Pstl, Xhol et EcoRl, et enfin 1156 pb de séquences situées en aval du COL C6L (« bras flanquant droit C6 »).

Le plasmide pMP528HRH (Perkus M. et al. J. Virol. 1989. 63. 3829-3836) a été utilisé comme matrice pour amplifier la séquence complète du promoteur vaccine H6 (N° d'accès GenBank M28351) avec les oligonucléotides suivants:
JCA291 (SEQ ID NO 15) (34 mer)
   5' AAACCCGGGTTCTTTATTCTATACTTAAAAAGTG 3'
et JCA292 (SEQ ID NO 16) (43 mer)
   5' AAAAGAATTCGTCGACTACGATACAAACTTAACGGATATCGCG 3'
pour amplifier un fragment ACP de 149 pb. Ce fragment a été digéré par les enzymes de restriction Smal et EcoRI pour isoler, après électrophorèse en gel d'agarose, un fragment de restriction SmaI-EcoRI de 138 pb. Ce fragment a alors été ligaturé avec le plasmide pC6L, préalablement digéré par Smal et EcoRI, pour donner le plasmide pJCA150.

### Exemple 8 : Construction du virus recombiné vCP1710 (canarypox virus recombiné exprimant le gène capside de la souche FCV 431)

Une réaction ACP a été réalisée en utilisant le plasmide p431-2-1 (exemple 5) comme matrice et les oligonucléotides suivants :
JCA293 (SEQ ID NO 17) (55 mer) :
et JCA294 (SEQ ID NO 18) (33 mer) :
   5' TTTTGTCGACTCATATTTTAACCATTCCACTCC 3'
pour amplifier un fragment ACP d'environ 2050 pb. Ce fragment a été digéré avec les enzymes de restriction Nrul et Sall pour isoler, après électrophorèse en gel d'agarose, le fragment Nrul-Sall de 2035 pb (fragment A). Le plasmide pJCA150 (exemple 7) a été digéré par les enzymes de restriction Nrul et Sall pour isoler, après électrophorèse en gel d'agarose, le fragment de restriction Nrul-Sall d'environ 4500 pb (fragment B). Les fragments A et B ont alors été ligaturés ensemble pour donner le plasmide pJCA152. Le plasmide pJCA152 a été linéarisé par Notl, puis transfecté dans des cellules primaires d'embryons de poulets infectées avec du canarypox virus (souche ALVAC) selon la technique de précipitation au phosphate de calcium précédemment décrite (Panicali et Paoletti Proc. Nat. Acad. Sci. 1982. 79. 4927-4931 ; Piccini et al. In Methods in Enzymology. 1987. 153. 545-563. Eds. Wu R. and Grossman L. Academic Press). Des plages positives ont été sélectionnées sur la base d'une hybridation avec une sonde radiomarquée spécifique du gène capside de la souche FCV 431. Ces plages ont subi 4 cycles successifs de sélection/purification de plages jusqu'à ce qu'une population pure ait été isolée. Une plage représentative de la recombinaison *in vitro* entre le plasmide donneur pJCA152 et le génome du canarypox virus ALVAC a alors été amplifiée et le stock de virus recombiné obtenu a été désigné vCP1710.

### Exemple 9 : Construction du virus recombiné vCP1711 1 (canarypox virus recombiné exprimant le gène capside de la souche FCV G1)

Une réaction ACP a été réalisée en utilisant le plasmide pG 1-4-5 (exemple 4) comme matrice et les oligonucléotides suivants :
JCA293 (SEQ ID NO 17)
et JCA295 (SEQ ID NO 19) (33 mer) :
   5' TTTTGTCGACTCATAGTTTTGTCATAGTACTCC 3'
pour amplifier un fragment ACP d'environ 2050 pb. Ce fragment a été digéré avec les enzymes de restriction Nrul et Sall pour isoler, après électrophorèse en gel d'agarose, un fragment Nrul-Sall de 2035 pb (fragment A). Le plasmide pJCA150 (exemple 7) a été digéré par les enzymes de restriction Nrul et Sall pour isoler, après électrophorèse en gel d'agarose, le fragment de restriction Nrul-Sall d'environ 4500 pb (fragment B). Les fragments A et B ont alors été ligaturés ensemble pour donner le plasmide pJCA153.

Le plasmide pJCA153 a été linéarisé par Notl, puis transfecté dans des cellules primaires d'embryons de poulets infectées avec du canarypox virus (souche ALVAC) selon la technique de précipitation au phosphate de calcium précédemment décrite (Panicali et Paoletti Proc. Nat. Acad. Sci. 1982. 79. 4927-4931 ; Piccini et al. In Methods in Enzymology. 1987. 153. 545-563. Eds. Wu R. and Grossman L. Academic Press). Des plages positives ont été sélectionnées sur la base d'une hybridation avec une sonde radiomarquée spécifique du gène capside de la souche FCV G 1. Ces plages ont subi 4 cycles successifs de sélection/purification de plages jusqu'à ce qu'une population pure ait été isolée. Une plage représentative de la recombinaison *in vitro* entre le plasmide donneur pJCA152 et le génome du canarypox virus ALVAC a alors été amplifiée et le stock de virus recombiné obtenu a été désigné vCP1711.

### Exemple 10 : Plasmide codant pour le GM-CSF félin

Du sang de chat a été récolté par prise de sang sur un tube contenant de l'EDTA. Les cellules mononucléées ont été récoltées par centrifugation sur un gradient de Ficoll, puis mises en culture en boîte de Petri de 60 mm de diamètre. Les cellules mononuclées de chat ont alors été stimulés avec de la concanavaline A (ConA) (concentration finale d'environ 4 µg/ml) et avec de la phyto-hémagglutinine (PHA) (concentration finale d'environ 10 µg/ml). Après stimulation, les lymphoblastes " ConA " et " PHA " ont été récoltés par grattage des boîtes de culture, et l'ARN total de ces cellules a été extrait en utilisant le kit " mRNA isolation kit for White Blood Cells " (Boehringer Mannheim/Roche Cat # 1 934 325).

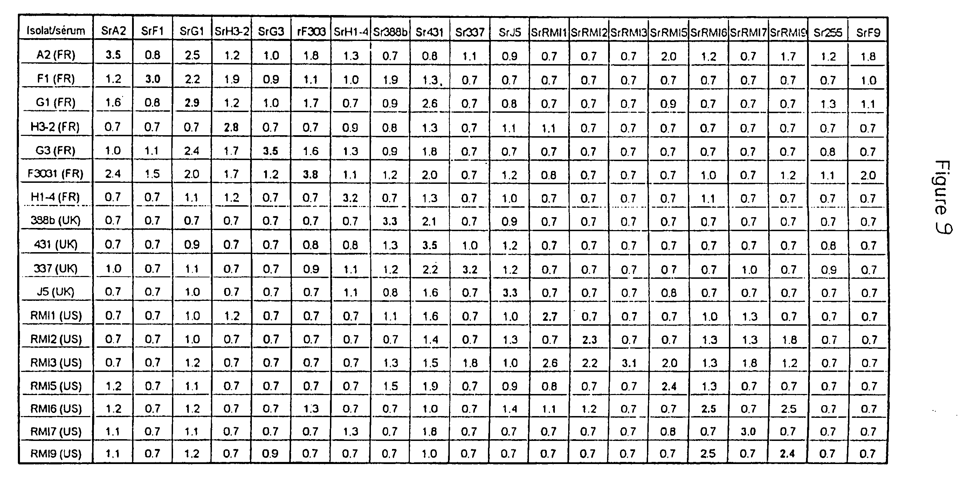

L'ARN total extrait des lymphoblastes de chat stimulés par la ConA et la PHA a servi de matrice pour la synthèse du premier brin d'ADN complémentaire. Ce premier brin d'ADN complémentaire a été produit par élongation de l'oligonucléotide p(dT)15 (Boehringer Mannheim/Roche Cat # 814 270). L'ADN complémentaire simple brin obtenu a été ensuite utilisé comme matrice pour une réaction d'ACP avec les oligonucléotides suivants :
JP578 (SEQ ID NO 20) (33 mer)
   5' TATGCGGCCGCCACCATGTGGCTGCAGAACCTG 3'
et JP579 (SEQ ID NO 21) (36 mer)
   5' TATGCGGCCGCTACGTATCACTTCTTGACTGGTTTC 3'
pour amplifier un fragment ACP d'environ 450 paires de bases (pb). Ce fragment a été digéré par Notl pour isoler, après électrophorèse en gel d'agarose, le fragment Notl-Notl de 450 pb. Ce fragment a été alors ligaturé avec le plasmide pVR1012 (Hartikka J. et al. Human Gene Therapy. 1997. 7. 1205-1217). Deux clones contenant la séquence GM-CSF félin (SEQ ID NO 22 et SEQ ID NO 23), dans la bonne orientation par rapport au promoteur hCMV/IE ont été identifiés respectivement pJP089 et pJP090. Ces deux plasmide ont une taille de 5364 pb (Figures 5 et 7).

La séquence du gène GM-CSF félin cloné dans le plasmide pJP089 contient 13 différences au niveau nucléotidique avec la séquence GM-CSF félin disponible sur GenBank (n° d'accès AF053007). Le changement le plus important est une changement C → T qui entraîne un changement Leucine → Phenylalanine pour l'acide aminé (première base du codon de l'acide aminé # 107 ; Figure 6). La séquence du gène GM-CSF félin cloné dans le plasmide pJP090 est équivalent à celle contenue dans le plasmide pJP089, sauf que le changement Leucine → Phenylalanine au n'existe pas pour l'acide aminé # 107 (Figure 8). La vérification de la séquence 3' du gène GM-CSF félin au moyen du kit 3' RACE a montré que, à cette position 107, on peut avoir chez le même chat, l'acide aminé Leucine ou l'acide aminé Phenylalanine.

### Exemple 11 : Fabrication des vaccins plasmidiques associés

Les divers plasmides nécessaires à la fabrication d'un vaccin associé sont mélangés. Ces plasmides peuvent être notamment ceux décrits dans les exemples 6 et 10 (pJCA151, pJP089 et pJP090) et les exemples 7 à 15 et 17 à 19 de la demande de brevet WO-A-9803660 (pPB179, pPB180, pPB181, pAB009, pAB053, pAB052, pAB056, pAB058, pAB029, pAB030, pAB083, pAB041). Les mélanges sont réalisés de telle manière que la concentration finale de chaque plasmide corresponde à la dose efficace de chaque plasmide. Les solutions utilisables pour ajuster la concentration finale du vaccin peuvent être soit une solution NaCl à 0,9 %, soit du tampon PBS.

### Exemple 12 : Formulation des plasmides vaccinaux

La solution d'ADN contenant un ou plusieurs plasmides selon l'exemple 11 est concentrée par précipitation éthanolique comme décrit dans Sambrook et al (1989). Le culot d'ADN est repris par une solution de NaCl 0.9% de façon à obtenir une concentration de 1 mg/ml. Une solution de DMRIE-DOPE à 0,75mM est préparée par reprise d'un lyophilisat de DMRIE-DOPE par un volume adapté d'H₂O stérile.

La formation des complexes ADN plasmidique-lipide est réalisée par dilution à parties égales de la solution de DMRIE-DOPE 0.75 mM par la solution d'ADN à 1 mg/ml dans NaCI 0.9%. La solution d'ADN. est introduire progressivement à l'aide d'une aiguille sertie 26G le long de la paroi du flacon contenant la solution de lipide cationique de façon à éviter la formation de mousse. On procède à une agitation douce dès que les deux solutions ont été mélangées. On obtient en final une composition comprenant 0,375 mM de DMRIE-DOPE et 500 µg/ml de plasmide.

Il est souhaitable que l'ensemble des solutions utilisées soient à température ambiante pour l'ensemble des opérations décrites ci-dessus. On laisse la complexation ADN/DMRIE-DOPE se mettre en place à température ambiante pendant 30 minutes avant de procéder à l'immunisation des animaux.

### Exemple 13 : Formulation des vecteurs canarypox vaccinaux

Pour la préparation de vaccins, les virus recombinés canarypox vCP1710 (exemple 8) et vCP1711 (exemple 9) peuvent être adjuvés avec des solutions de carbomère. Le carbomère préféré est le Carbopol^{™} 974P fabriqué par BF Goodrich, Ohio, USA (poids moléculaire d'environ 3 000 000).

Une solution stock à 1,5 % de Carbopol^{™} 974P est initialement préparée dans de l'eau distillée contenant 1 g/l de chlorure de sodium. Cette solution stock est alors utilisée pour l'élaboration d'une solution à 4 mg/ml de Carbopol^{™} 974P en eau physiologique. La solution stock est mélangée au volume adéquat d'eau physiologique, soit en une étape unique soit en plusieurs étapes successives, la valeur du pH est ajustée à chaque étape avec une solution d'hydroxyde de sodium 1N (ou encore plus concentrée) afin d'obtenir une valeur finale de pH de 7,3-7,4.

La solution de Carbopol^{™} 974P prête à l'emploi ainsi obtenue peut être utilisée pour reprendre des virus recombinés lyophilisés (e.g. vCP1710, vCP1711) ou pour diluer des solutions stocks concentrées de virus recombinés (e.g. vCP1710, vCP1711). Par exemple, pour obtenir une suspension virale contenant 10⁸ pfu par dose de 1 ml, on peut diluer une solution virale stock de manière à obtenir un titre de 10^{8,3} pfu/ml, puis diluer à parties égales avec ladite solution de Carbopol^{™} 974P prête à l'emploi à 4 mg/ml.

### Exemple 14 : Tests d'immunofluorescence indirecte (IFI)

Les tests IFI sont effectués sur des plaques 96 puits contenant les cellules CRFK cultivées en monocouches et infectées par les virus FCV à tester.

200 µl par cupule d'une suspension de cellules CRFK à 90 000 cellules/ml en milieu F15 (Gibco BRL, Cat # 045-1075) contenant 5 % de sérum de veau foetal sont mises en culture en plaque de 96 cupules. A la confluence, 320 DICC50 de FCV sont inoculés sous 100 µl de milieu F15. Lorsque les premiers foyers d'ECP apparaissent, les cellules sont alors rincées par du PBS sans calcium ni magnésium (PBS', Sigma) froid, puis fixées à -20°C pendant 30 minutes par de l'acétone froid contenant 5 % v/v d'eau. Après séchage, les cellules infectées et fixées sont mises en contact pendant 30 minutes à 37°C avec 100 µl par cupule de liquide d'ascite correspondant à l'anticorps monoclonal 44 anti-FCV 431 (hybridome 431 2 0 17 E9 T, déposé à la CNCM sous le numéro d'accès I-2282), dilué au 1/5000 ème environ dans du tampon TRIS-HCl 50 mM pH7,6.

Après deux rinçages en PBS, la fixation des anticorps est révélée par incubation dans les mêmes conditions d'un anticorps de chèvre anti-IgG de souris conjugué à de l'isothianate de fluorescéine (Biosys, conjugué FITC à 2mg/ml) et dilué à 1/150 en tampon TRIS-HCl 50 mM pH7,6. La lecture est faite sous microscope optique en lumière UV.

Cet anticorps monoclonal a été testé vis-à-vis de chacun des isolats du panel. Il s'est fixé exclusivement sur les cellules CRFK infectées par le FCV 431.

Ce test peut être utilisé pour déterminer les équivalents de la souche FCV 431. Ces équivalents sont ceux sur lesquels se fixe l'anticorps monoclonal 44.

### Exemple 15 : Séroneutralisation croisée in vitro

Des tests de séroneutralisation croisée ont été effectués entre 18 isolats de terrain obtenus par écouvillonnages pharyngés pratiqués sur des chats présentant des signes de calicivirose féline. 7 ont pour origine géographique la France, il s'agit des isolats identifiés A2, F3031, G1, G3, F1, H3-2 et H1-4. 4 ont pour origine géographique la Grande-Bretagne, il s'agit des isolats identifiés J5, 337, 388b et 431. Enfin, 7 ont pour origine géographique les USA, il s'agit des isolats identifiés RMI1, RMI2, RMI3, RMI5, RMI6, RMI7 et RMI9.

Pour chaque virus FCV, un antisérum a été produit par inoculation de chatons par la voie oronasale avec 10^{6.0} DICC₅₀ du virus FCV concerné. Les chatons exempts de pathogènes spécifiques (EOPS) étaient âgés de 10 à 14 semaines.

Le sérum de chaque animal a été collecté un mois après l'infection. Les sérums ont été inactivés par la chaleur (30 minutes à 56°C), répartis, aliquotés et stockés à -20°C.

Le sérum obtenu pour chaque isolat a été testé pour son aptitude à neutraliser les 18 isolats. Les sérums ont été dilués en cascade au tiers avec du milieu DMEM dans des plaques à 96 puits pour culture cellulaire. A 0,05 ml du sérum dilué on a ajouté 0,05 ml de milieu de culture contenant approximativement 100 DICC₅₀ de la souche virale sélectionnée. Ce mélange a été incubé pendant 2 heures à 37°C en étuve en atmosphère contenant 5% CO₂.

0,15 ml d'une suspension de cellules CRFK contenant environ 100 000 cellules par ml a été ensuite ajoutée à chaque mélange. L'effet cytopathique a été observé par microscopie à contraste de phase après 4 jours de culture à 37°C en atmosphère contenant 5% CO₂. Les titres neutralisants de chaque sérum ont été calculés d'après la méthode de Kärber. Les titres sont donnés sous la forme de la dilution la plus importante inhibant l'effet cytopathique pour 50 % des cupules. Les titres sont exprimés en log₁₀. Le titre minimum ainsi trouvé a été de 0,7 log₁₀ VN₅₀. Chaque sérum a été titré au moins deux fois, de préférence trois fois.

La Figure 9 donne l'ensemble des titrés neutralisants obtenus lors des séroneutralisations croisées effectuées entre ces 18 souches FCV et ces 18 sérums.

## Revendications

1. Fragment d'acide nucléique comprenant la séquence nucléotidique représentée à la SEQ ID NO:6.

2. Fragment d'acide nucléique de la séquence nucléotidique représentée à la SEQ ID NO:6 codant pour un épitope, peptide ou polypeptide, ledit épitope, peptide ou polypeptide étant capable d'induire *in vivo* chez le chat des anticorps ayant le spectre de neutralisation croisée de l'antisérum de la souche FCV 431 telle que déposée à la CNCM sous le numéro d'accès I-2166.

3. Fragment d'acide nucléique codant pour la protéine de capside représentée à la SEQ ID NO : 7.

4. Fragment d'acide nucléique codant pour un fragment immunologiquement actif de la protéine de capside représentée à la SEQ ID NO : 7, ledit fragment immunologiquement actif étant capable d'induire *in vivo* chez le chat des anticorps ayant le spectre de neutralisation croisée de l'antisérum de la souche FCV 431 telle que déposée à la CNCM sous le numéro d'accès I-2166.

5. Fragment d'acide nucléique codant pour la protéine de capside d'un calicivirus félin (FCV) reconnu par l'anticorps monoclonal 44 secrété par l'hybridome déposé à la CNCM sous le numéro d'accès I-2282, ladite protéine de capside étant capable d'induire *in vivo* chez le chat des anticorps ayant le spectre de neutralisation croisée de l'antisérum de la souche FCV 431 telle que déposée à la CNCM sous le numéro d'accès I-2166.

6. Fragment d'acide nucléique codant pour un fragment immunologiquement actif de la protéine de capside d'un calicivirus félin (FCV) reconnu par l'anticorps monoclonal 44 secrété par l'hybridome déposé à la CNCM sous le numéro d'accès 1-2282, ledit fragment immunologiquement actif.

7. Vecteur d'expression *in vivo* ou *in vitro* comprenant un fragment d'acide nucléique selon l'une des revendications 1 à 6.

8. Vecteur d'expression *in vivo* ou *in vitro* selon la revendication 7, dans lequel le fragment d'acide nucléique est sous la dépendance de signaux régulateurs de la transcription.

9. Vecteur d'expression *in vivo* ou *in vitro* selon la revendication 7 ou 8, choisi dans le groupe constitué des poxvirus, adénovirus, herpèsvirus et baculovirus.

10. Vecteur d'expression *in vivo* ou *in vitro* selon la revendication 7 ou 8, le vecteur d'expression étant un plasmide.

11. Vecteur d'expression *in vivo* ou *in vitro* selon l'une quelconque des revendications 7 à 10, qui comprend en outre un fragment d'acide nucléique comprenant tout ou partie de la séquence nucléotidique représentée à la SEQ ID NO:4.

12. Vecteur d'expression *in vivo* ou *in vitro* selon l'une quelconque des revendications 7 à 10, qui comprend un fragment d'acide nucléique additionnel, le fragment d'acide nucléique additionnel comprenant la séquence nucléotidique représentée à la SEQ ID NO: 4.

13. Vecteur d'expression *in vivo* ou *in vitro* selon l'une quelconque des revendications 7 à 10, qui comprend un fragment d'acide nucléique additionnel, le fragment d'acide nucléique additionnel comprenant un fragment de la séquence nucléotidique représentée à la SEQ ID NO: 4 codant pour un épitope, peptide ou polypeptide capable d'induire *in vivo* chez le chat des anticorps ayant le spectre de neutralisation croisée de l'antisérum de la souche FCV G1 telle que déposée à la CNCM sous le numéro d'accès I-2167.

14. Vecteur d'expression *in vivo* ou *in vitro* selon l'une quelconque des revendications 7 à 10, qui comprend un fragment d'acide nucléique additionnel, le fragment d'acide nucléique additionnel codant pour la protéine de capside représentée à la SEQ ID NO: 5.

15. Vecteur d'expression *in vivo* ou *in vitro* selon l'une quelconque des revendications 7 à 10, qui comprend un fragment d'acide nucléique additionnel, le fragment d'acide nucléique additionnel codant pour un fragment immunologiquement actif de la protéine de capside représentée à la SEQ ID NO: 5, ledit fragment immunologiquement actif étant capable d'induire *in vivo* chez le chat des anticorps ayant le spectre de neutralisation croisée de l'antisérum de la souche FCV G1 telle que déposée à la CNCM sous le numéro d'accès I-2167.

16. Vecteur d'expression *in vivo* ou *in vitro* selon l'une quelconque des revendications 12 to 15, dans lequel le fragment d'acide nucléique est sous la dépendance de signaux régulateurs de la transcription.

17. Vecteur d'expression selon l'une quelconque des revendications 7 à 16, comprenant en outre une séquence nucléotidique d'un immunogène d'un autre pathogène félin.

18. Vecteur d'expression selon la revendication 17, dans lequel l'autre pathogène félin est choisi dans le groupe consistant en le virus de la rhinotrachéite féline (nommé aussi herpèsvirus félin, FHV), le virus de la leucémie féline (FeLV), le parvovirus félin (FPV), le virus de la péritonite infectieuse féline (FIPV), le virus de l'immunodéficience féline (FIV), le virus de la rage, *Chlamydia.*

19. Préparation immunogène ou vaccin contre la calicivirose féline, comprenant un vecteur d'expression *in vivo* selon l'une des revendications 7 à 18, et un véhicule ou excipient acceptable sur le plan vétérinaire.

20. Préparation immunogène ou vaccin contre la calicivirose féline et contre au moins un autre pathogène félin, comprenant un premier vecteur d'expression *in vivo* selon l'une des revendications 7 à 18 et un second vecteur d'expression *in vivo* dans lequel est insérée une séquence codant pour un immunogène d'un autre pathogène félin.

21. Préparation immunogène ou vaccin multivalent contre la calicivirose féline et contre au moins un autre pathogène félin, comprenant un vecteur d'expression *in vivo* selon la revendication 17 ou 18, et un véhicule ou excipient acceptable sur le plan vétérinaire.

22. Préparation immunogène ou vaccin selon l'une quelconque des revendications 19 à 21, dans laquelle l'un au moins des vecteurs d'expression *in vivo* est un poxvirus.

23. Préparation immunogène ou vaccin selon la revendication 22, dans laquelle le poxvirus est un canarypox.

24. Préparation immunogène ou vaccin selon l'une quelconque des revendications 19 à 23, comprenant en outre un adjuvant.

25. Préparation immunogène ou vaccine selon la revendication 24, dans laquelle l'un au moins des vecteurs d'expression *in vivo* est un plasmide et en ce que l'adjuvant est un lipide cationique contenant un sel d'ammonium quaternaire, de formule: 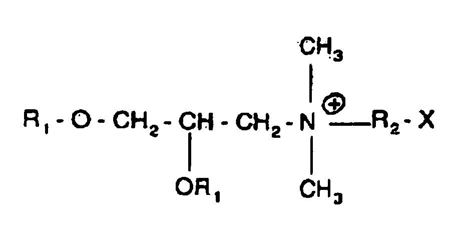 dans laquelle R1 est un radical aliphatique linéaire, saturé ou insaturé, ayant de 12 à 18 atomes de carbone, R2 est un autre radical aliphatique, renfermant 2 ou 3 atomes de carbone, et X un groupement hydroxyle ou amine.

26. Préparation immunogène ou vaccin selon la revendication 25, dans laquelle l'adjuvant est le DMRIE.

27. Préparation immunogène ou vaccin selon l'une ou l'autre des revendications 25 et 26, dans laquelle l'adjuvant est couplé avec un lipide neutre.

28. Préparation immunogène ou vaccin selon la revendication 27, dans laquelle le lipide neutre comprend le DOPE.

29. Préparation immunogène ou vaccin selon la revendication 27, dans laquelle l'adjuvant comprend le DMRIE-DOPE.

30. Préparation immunogène ou vaccin selon la revendication 22 ou 23, qui comprend en outre un adjuvant comprenant un polymère de l'acide acrylique ou méthacrylique.

31. Préparation immunogène ou vaccin selon la revendication 24, dans laquelle l'adjuvant comprend un carbomère.

32. Préparation immunogène ou vaccin selon l'une quelconque des revendications 19 à 31, comprenant en outre un vecteur plasmidique d'expression *in vivo* dans lequel est inséré le gène codant pour le GM-CSF félin.

33. Préparation immunogène ou vaccin contre la calicivirose féline comprenant la protéine de capside de la souche FCV 431 assemblée sous forme de capsides vides, cette protéine ayant la séquence SEQ ID NO:7.

34. Préparation immunogène ou vaccin contre la calicivirose féline comprenant un fragment ou épitope immunologiquement actif de la séquence SEQ ID NO:7, ledit fragment ou épitope immunologiquement actif étant capable d'induire *in vivo* chez le chat des anticorps ayant le spectre de neutralisation croisée de l'antisérum de la souche FCV 431 telle que déposée à la CNCM sous le numéro d'accès I-2166.

35. Préparation immunogène ou vaccin contre la calicivirose féline, comprenant la protéine de capside d'un calicivirus félin reconnu par l'anticorps monoclonal 44, assemblée sous forme de capsides vides.

36. Préparation immunogène ou vaccin contre la calicivirose féline, comprenant un fragment ou épitope immunologiquement actif de la protéine de capside d'un calicivirus félin reconnu par l'anticorps monoclonal 44, ledit fragment ou épitope immunologiquement actif étant capable d'induire *in vivo* chez le chat des anticorps ayant le spectre de neutralisation croisée de l'antisérum de la souche FCV 431 telle que déposée à la CNCM sous le numéro d'accès I-2166.

37. Préparation immunogène ou vaccin selon l'une quelconque des revendications 33 to 36, comprenant en outre la protéine de capside de la souche FCV G1 assemblée sous forme de capsides vides, cette protéine ayant la séquence SEQ ID NO: 5.

38. Préparation immunogène ou vaccin selon l'une quelconque des revendications 33 to 36, comprenant en outre un fragment ou épitope immunologiquement actif de la protéine ayant la séquence SEQ ID NO: 5, ledit fragment ou épitope immunologiquement actif étant capable d'induire *in vivo* chez le chat des anticorps ayant le spectre de neutralisation croisée de l'antisérum de la souche FCV G1 telle que déposée à la CNCM sous le numéro d'accès I-2167.

39. Protéine de capside isolée, purifiée ou synthétique, ayant la séquence SEQ ID NO: 7 assemblée sous forme de capsides vides.

40. Fragment ou épitope immunologiquement actif de la protéine ayant la séquence SEQ ID NO: 7, ledit fragment ou épitope immunologiquement actif étant capable d'induire *in vivo* chez le chat des anticorps ayant le spectre de neutralisation croisée de l'antisérum de la souche FCV 431 telle que déposée à la CNCM sous le numéro d'accès I-2166.

41. Protéine de capside isolée, purifiée ou synthétique, d'un calicivirus félin reconnu par l'anticorps monoclonal 44, assemblée sous forme de capsides vides, ladite protéine de capside étant capable d'induire *in vivo* chez le chat des anticorps ayant le spectre de neutralisation croisée de l'antisérum de la souche FCV 431 telle que déposée à la CNCM sous le numéro d'accès I-2166.

42. Fragment ou épitope immunologiquement actif de la protéine de capside isolée, purifiée ou synthétique, d'un calicivirus félin reconnu par l'anticorps monoclonal 44, ledit fragment ou épitope immunologiquement actif étant capable d'induire *in vivo* chez le chat des anticorps ayant le spectre de neutralisation croisée de l'antisérum de la souche FCV 431 telle que déposée à la CNCM sous le numéro d'accès I-2166.

43. Préparation immunogène ou vaccin, comprenant une protéine de capside isolée, purifiée ou synthétique ou fragment ou épitope immunologiquement actif selon l'une quelconque des revendications 39 à 42, et un véhicule ou excipient acceptable sur le plan vétérinaire.

44. Préparation immunogène ou vaccin selon la revendication 43, comprenant en outre un immunogène d'un autre pathogène félin ou un vecteur d'expression comprenant une séquence nucléotidique d'un immunogène d'un autre pathogène félin.

45. Préparation immunogène ou vaccin selon la revendication 44, dans laquelle l'autre pathogène félin est choisi dans le groupe consistant en le virus de la rhinotrachéite féline (nommé aussi herpèsvirus félin, FHV), le virus de la leucémie féline (FeLV), le parvovirus félin (FPV), le virus de la péritonite infectieuse féline (FIPV), le virus de l'immunodéficience féline (FIV), le virus de la rage, *Chlamydia.*

46. Préparation immunogène ou vaccin selon l'une quelconque des revendications 43 à 45, comprenant en outre la protéine de capside isolée, purifiée ou synthétique, ayant la séquence SEQ ID NO: 5.

47. Préparation immunogène ou vaccin selon l'une quelconque des revendications 43 à 45, comprenant en outre un fragment ou épitope immunologiquement actif de la protéine ayant la séquence SEQ ID NO: 5, ledit fragment ou épitope immunologiquement actif étant capable d'induire *in vivo* chez le chat des anticorps ayant le spectre de neutralisation croisée de l'antisérum de la souche FCV G1 telle que déposée à la CNCM sous le numéro d'accès I-2167..

48. Préparation immunogène ou vaccin selon l'une quelconque des revendications 43 à 45, comprenant en outre un vecteur d'expression comprenant une séquence nucléotidique codant pour la protéine ayant la séquence SEQ ID NO: 5.

49. Préparation immunogène ou vaccin selon l'une quelconque des revendications 43 à 45, comprenant en outre un vecteur d'expression comprenant une séquence nucléotidique codant pour un fragment ou épitope immunologiquement actif de la protéine ayant la séquence SEQ ID NO: 5, ledit fragment ou épitope immunologiquement actif étant capable d'induire *in vivo* chez le chat des anticorps ayant le spectre de neutralisation croisée de l'antisérum de la souche FCV G1 telle que déposée à la CNCM sous le numéro d'accès 1-2167.

## Claims

1. A nucleic acid fragment comprising the nucleotide sequence represented in SEQ ID NO: 6.

2. A nucleic acid fragment of the nucleotide sequence represented in SEQ ID NO: 6 encoding an epitope, peptide or polypeptide, said epitope, peptide or polypeptide being capable of inducing *in vivo* in cats antibodies having the cross-neutralization spectrum of the antiserum against FCV 431 strain deposited at the CNCM under the accession number I-21666.

3. A nucleic acid fragment encoding the capsid protein represented in SEQ ID NO:7.

4. A nucleic acid fragment encoding an immunologically active fragment of the capsid protein represented in SEQ ID NO:7, said immunologically active fragment being capable of inducing *in vivo* in cats antibodies having the cross-neutralization spectrum of the antiserum against the FCV 431 strain deposited at the CNCM under the accession number I-2166.

5. A nucleic acid fragment encoding Feline calicivirus (FCV) capsid protein of an FCV recognized by the monoclonal antibody 44 secreted by the hybridoma deposited at the CNCM under the accession number 1-2282, said capsid protein being capable of inducing *in vivo* in cats antibodies having the cross-neutralization spectrum of the antiserum against the FCV 431 strain deposited at the CNCM under the accession number I-2166.

6. A nucleic acid fragment encoding an immunologically active fragment of Feline calicivirus (FCV) capsid protein recognized by the monoclonal antibody 44 secreted by the hybridoma deposited at the CNCM under the accession number 1-2282, said immunologically active fragment being capable of inducing *in vivo* in cats antibodies having the cross-neutralization spectrum of the antiserum against the FCV 431 strain deposited at the CNCM under the accession number I-2166.

7. *In vivo* or *in vitro* expression vector comprising a nucleic acid fragment according to any one of claims 1 to 6.

8. *In vivo* or *in vitro* expression vector according to claim 7, wherein the nucleic acid fragment is under the control of a transcription regulation signal.

9. *In vivo* or *in vitro* expression vector according to claim 7 or 8, wherein the vector is a poxvirus, an adenovirus, a herpesvirus or a baculovirus.

10. *In vivo* or *in vitro* expression vector according to claim 7 or 8, wherein the vector is a plasmid.

11. *In vivo* or *in vitro* expression vector according to any one of claims 7 to 10, wherein the vector comprises, in addition, a DNA fragment comprising all or part of the nucleotide sequence represented in SEQ ID NO:4.

12. *In vivo* or *in vitro* expression vector according to any one of claims 7 to 10, wherein the vector comprises an additional nucleic acid fragment, and the additional nucleic acid fragment comprises the nucleotide sequence represented in SEQ ID NO:4.

13. *In vivo* or *in vitro* expression vector according to any one of claims 7 to 10, wherein the vector comprises an additional nucleic acid fragment, and the additional nucleic acid fragment comprises a fragment of the nucleotide sequence represented in SEQ ID NO:4 encoding an epitope, peptide or polypeptide capable of inducing *in vivo* in cats antibodies having the cross-neutralization of the antiserum against the FCV G1 strain deposited at the CNCM under the accession number I-2167.

14. *In vivo* or *in vitro* expression vector according to any one of claims 7 to 10, wherein the vector comprises an additional nucleic acid fragment, and the additional nucleic acid fragment encodes the capsid protein represented in SEQ ID NO:5.

15. *In vivo* or *in vitro* expression vector according to any one of claims 7 to 10, wherein the vector comprises an additional nucleic acid fragment, and the additional nucleic acid fragment encodes an immunologically active fragment of the capsid protein represented in SEQ ID NO:5, said immunologically active fragment being capable of inducing *in vivo* in cats antibodies having the cross-neutralization of the antiserum against the FCV G1 strain deposited at the CNCM under the accession number I-.

16. The *in vivo* or *in vitro* expression vector according to any one of claims 12 to 15, wherein the additional nucleic acid fragment is under the control of a transcription regulation signal.

17. Expression vector according to any one of claims 7 to 16, wherein the vector comprises, in addition, a nucleotide sequence of an immunogen of another feline pathogen.

18. Expression vector according to claim 17, wherein the other feline pathogen is feline rhinotrachitis virus (also called feline herpesvirus, FHV), the feline leukemia virus (FeLV), the feline parvoviruses (FPV), the feline infectious peritonitis virus (FIPV), the feline immunodeficiency virus (FIV), the rabies virus, or *Chlamydia.*

19. Immunogenic preparation or vaccine wherein the preparation or vaccine comprises an *in vivo* expression vector according to one of claims 7 to 18, and a veterinarily acceptable vehicle or excipient.

20. Immunogenic preparation or vaccine against feline calicivirus and against at least one other feline pathogen, comprising a first *in vivo* expression vector according to one of claims 7 to 18 and a second *in vivo* expression vector into which a nucleotide sequence encoding an immunogen of an additional feline pathogen has been inserted.

21. Multivalent immunogenic preparation or vaccine against feline calicivirus and against at least one other feline pathogen, comprising an *in vivo* expression vector according to claim 17 or 18, and a veterinarily acceptable vehicle or excipient.

22. Immunogenic preparation or vaccine according to any one of claims 19 to 21, wherein at least one of the *in vivo* expression vectors is a poxvirus.

23. Immunogenic preparation or vaccine according to claim 22 wherein the poxvirus is canarypox.

24. Immunogenic preparation or vaccine according to any one of claims 19 to 23, additionally comprising an adjuvant.

25. Immunogenic preparation or vaccine according to claim 24, wherein at least one of the *in vivo* expression vectors is a plasmid and the adjuvant is a cationic lipid containing a quaternary ammonium salt of formula: 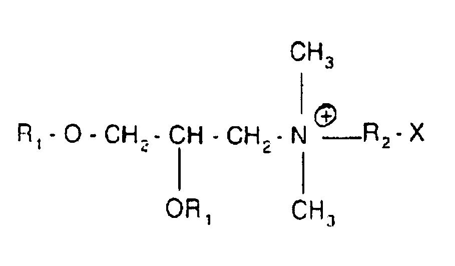 in which R1 is a saturated or unsaturated, linear aliphatic radical having from 12 to 18 carbon atoms, R2 is another aliphatic radical containing 2 or 3 carbon atoms, and X is a hydroxyl or amine group.

26. Immunogenic preparation or vaccine according to claim 25, wherein the adjuvant is DMRIE.

27. Immunogenic preparation or vaccine according to claim 25 or 26, wherein the adjuvant is coupled with a neutral lipid.

28. Immunogenic preparation or vaccine according to claim 27, wherein the neutral lipid comprises DOPE.

29. Immunogenic preparation or vaccine according to claim 27, wherein the adjuvant comprises DMRIE-DOPE.

30. Immunogenic preparation or vaccine according to claim 22 or 23, wherein the preparation or vaccine additionally comprises an adjuvant comprising a polymer of acrylic or methacrylic acid.

31. Immunogenic preparation or vaccine of claim 24, wherein the adjuvant comprises a carbomer.

32. Immunogenic preparation or vaccine according to any one of claims 19 to 31, comprising an *in vivo* expression plasmid vector into which the gene encoding feline GM-CSF is inserted.

33. Immunogenic preparation or vaccine against FCV, comprising a capsid protein of the FCV 431 strain assembled in the form of empty capsids, the capsid protein having the sequence SEQ ID NO: 7.

34. Immunogenic preparation or vaccine against FCV, comprising an immunogically active fragment or epitope of SEQ ID NO:7, said immunologically active fragment or epitope being capable of inducing *in vivo* in cats antibodies the cross-neutralization of the antiserum against the FCV 431 strain deposited at the CNCM under the accession number I-2166.

35. Immunogenic preparation or vaccine against FCV, comprising a FCV capsid protein of a FCV being recognized by the monoclonal antibody 44 assembled in the form of empty capsids.

36. Immunogenic preparation or vaccine against FCV, comprising an immunologically active fragment or epitope of a FCV capsid protein of an FCV being recognized by the monoclonal antibody 44, said immunologically active fragment or epitope being capable of inducing *in vivo* in cats antibodies having the cross-neutralization of the antiserum against the FCV 431 strain deposited at the CNCM under the accession number I-2166.

37. Immunogenic preparation or vaccine, according to any one of claims 33 to 36, comprising, in addition, a capsid protein of the FCV G1 strain, assembled in the form of empty capsids, the capsid protein having the sequence SEQ ID NO:5.

38. Immunogenic preparation or vaccine according to any one of claims 33 to 36, comprising, in addition, an immunologically active fragment or epitope of SEQ ID NO:5, said immunologically active fragment or epitope being capable of inducing *in vivo* in cats antibodies having the cross-neutralization of the antiserum against the FCV G1 strain deposited at the CNCM under the accession number I-2167.

39. Isolated, purified or synthetic capsid protein having the sequence SEQ ID NO:7 assembled in the form of empty capsids.

40. An immunologically active fragment or epitope of the sequence SEQ ID NO:7, said immunologically active fragment or epitope being capable of inducing *in vivo* in cats antibodies having the cross-neutralization spectrum of the antiserum against the FCV 431 strain deposited at the CNCM under the accession number I-2166.

41. Isolated, purified or synthetic FCV capsid protein recognized by the monoclonal antibody 44 assembled in the form of empty capsids, said capsid protein being capable of inducing *in vivo* in cats antibodies having the cross-neutralization spectrum of the antiserum against the FCV 431 strain deposited at the CNCM under the accession number I-2166.

42. An immunologically active fragment or epitope of an isolated, purified or synthetic FCV capsid protein recognized by the monoclonal antibody 44, said immunologically active fragment or epitope being capable of inducing *in vivo* in cats antibodies having the cross-neutralization spectrum of the antiserum against the FCV 431 strain deposited at the CNCM under the accession number I-2166.

43. Immunogenic preparation or vaccine comprising an isolated, purified or synthetic protein or an immunologically active fragment or epitope of any one of claims 39 to 42 and a veterinarily acceptable carrier.

44. Immunogenic preparation or vaccine according to claim 43, comprising, in addition, an immunogen of another feline pathogen, or an expression vector containing a nucleotide sequence of an immunogen of another feline pathogen.

45. Immunogenic preparation or vaccine according to claim 44, wherein the other feline pathogen is feline rhinotrachitis virus (also called feline herpesvirus, FHV), the feline leukemia virus (FeLV), the feline parvoviruses (FPV), the feline infectious peritonitis virus (FIPV), the feline immunodeficiency virus (FIV), the rabies virus, or *Chlamydia.*

46. Immunogenic preparation or vaccine of any one of claims 43 to 45 further comprising an isolated, purified or synthetic capsid protein having the sequence SEQ ID NO:5.

47. Immunogenic preparation or vaccine of any one of claims 43 to 45 further comprising an, or an isolated, purified or synthetic protein comprising an immunologically active fragment or epitope of SEQ ID NO:5, said immunologically active fragment or epitope being capable of inducing *in vivo* in cats antibodies having the cross-neutralization of the antiserum against the FCV G1 strain deposited at the CNCM under the accession number 1-2167.

48. Immunogenic preparation or vaccine of any one of claims 43 to 45 further comprising an expression vector that contains a nucleic acid molecule encoding the protein having the sequence SEQ ID NO:5.

49. Immunogenic preparation or vaccine of any one of claims 43 to 45 further comprising an expression vector that contains a nucleic acid molecule encoding an immunologically active fragment or epitope of SEQ 10 NO:5, said immunologically active fragment or epitope being capable of inducing *in vivo* in cats antibodies having the cross-neutralization of the antiserum against the FCV G1 strain deposited at the CNCM under the accession number I-2167.

## Patentansprüche

1. Nucleinsäurefragment, das die in SEQ ID NO:6 dargestellte Nucleotidsequenz umfasst.

2. Nucleinsäurefragment der in SEQ ID NO:6 dargestellten Nucleotidsequenz, das ein Epitop, Peptid oder Polypeptid codiert, wobei das Epitop, Peptid oder Polypeptid in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

3. Nucleinsäurefragment, das das in SEQ ID NO:7 dargestellte Kapsidprotein codiert.

4. Nucleinsäurefragment, das ein immunologisch aktives Fragment des in SEQ ID NO:7 dargestellten Kapsidproteins codiert, wobei das immunologisch aktive Fragment in der Lage ist, bei der Katze *in vivo* Antikörper induzieren, die das Kreuzneutralisationsspektrum des Antiserums des Stamms FCV 431, wie beim CNCM unter der Hinterlegungsnummer I-2166 hinterlegt, haben.

5. Nucleinsäurefragment, das das Kapsidprotein eines felinen Calicivirus (FCV) codiert, das von dem monoclonalen Antikörper 44 erkannt wird, der durch das beim CNCM unter der Hinterlegungsnummer 1-2282 hinterlegte Hybridom sezerniert wird, wobei das Kapsidprotein in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

6. Nucleinsäurefragment, das ein immunologisch aktives Fragment des Kapsidproteins eines felinen Calicivirus (FCV) codiert, das von dem monoclonalen Antikörper 44 erkannt wird, der durch das beim CNCM unter der Hinterlegungsnummer I-2282 hinterlegte Hybridom sezerniert wird, wobei das immunologisch aktive Fragment in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

7. *In vivo-* oder *in vitro*-Expressionsvektor, der ein Nucleinsäurefragment nach einem der Ansprüche 1 bis 6 umfasst.

8. *In vivo-* oder *in vitro*-Expressionsvektor nach Anspruch 7, in dem ein Nucleinsäurefragment unter der Kontrolle von Regulationssignalen der Transkription steht.

9. *In vivo-* oder *in vitro*-Expressionsvektor nach Anspruch 7 oder 8, ausgewählt aus der Gruppe bestehend aus Pockenvirus, Adenovirus, Herpesvirus oder Baculovirus.

10. *In vivo-* oder *in vitro*-Expressionsvektor nach Anspruch 7 oder 8, wobei der Expressionsvektor ein Plasmid ist.

11. *In vivo-* oder *in vitro*-Expressionsvektor nach einem der Ansprüche 7 bis 10, der des Weiteren ein Nucleinsäurefragment umfasst, das die ganze oder einen Teil der in SEQ ID NO:4 dargestellten Nucleotidsequenz umfasst.

12. *In vivo-* oder *in vitro*-Expressionsvektor nach einem der Ansprüche 7 bis 10, der des Weiteren ein zusätzliches Nucleinsäurefragment umfasst, wobei das zusätzliche Nucleinsäurefragment die in SEQ ID NO:4 dargestellte Nucleotidsequenz umfasst.

13. *In vivo-* oder *in vitro*-Expressionsvektor nach einem der Ansprüche 7 bis 10, der des Weiteren ein zusätzliches Nucleinsäurefragment umfasst, wobei das zusätzliche Nucleinsäurefragment ein Fragment der in SEQ ID NO:4 dargestellten Nucleotidsequenz umfasst, das ein Epitop, Peptid oder Polypeptid codiert, wobei das Epitop, Peptid, oder Polypeptid in der Lage ist, bei der Katze *in vivo* Antikörper zu induzieren, die das Kreuzneutralisationsspektrum des Antiserums des Stamms FCV G1, wie beim CNCM unter der Hinterlegungsnummer I-2167 hinterlegt, haben.

14. *In vivo-* oder *in vitro*-Expressionsvektor nach einem der Ansprüche 7 bis 10, der des Weiteren ein zusätzliches Nucleinsäurefragment umfasst, wobei das zusätzliche Nucleinsäurefragment das in SEQ ID NO:5 dargestellte Proteinkapsid codiert.

15. *In vivo-* oder *in vitro*-Expressionsvektor nach einem der Ansprüche 7 bis 10, der ein zusätzliches Nucleinsäurefragment umfasst, wobei das zusätzliche Nucleinsäurefragment ein immunologisch aktives Fragment des in SEQ ID NO:5 dargestellten Proteinkapsids codiert, wobei das immunologisch aktive Fragment in der Lage ist, bei der Katze *in vivo* Antikörper zu induzieren, die das Kreuzneutralisationsspektrum des Antiserums des Stamms FCV G1, wie beim CNCM unter der Hinterlegungsnummer I-2167 hinterlegt, haben.

16. *In vivo-* oder *in* v*itro*-Expressionsvektor nach einem der Ansprüche 12 bis 15, in dem ein Nucleinsäurefragment von Regulationssignalen der Transkription abhängig ist.

17. Expressionsvektor nach einem der Ansprüche 7 bis 16, der des Weiteren eine Nucleotidsequenz eines Immunogens eines anderen felinen Pathogens umfasst.

18. Expressionsvektor nach Anspruch 17, bei dem das andere feline Pathogen ausgewählt ist aus der Gruppe bestehend aus feliner viraler Rhinoracheitis (auch felines Herpesvirus, FHV, genannt), Felinem Läukemievirus (FeLV), Felinem Parvovirus (FPV), Felinem Infektiösem Peritonitis Virus (FIPV), Felinem Immundefizienz-Virus (FIV), Tollwut, *Chlamydia.*

19. Immunogene Zubereitung oder Impfstoff gegen felines Calicivirus, umfassend einen *in vivo-* oder *in vitro-*Expressionsvektor nach einem der Ansprüche 7 bis 18, und ein aus veterinärer Sicht verträglicher Träger oder Exzipient.

20. Immunogene Zubereitung oder Impfstoff gegen felines Calicivirus und gegen mindestens ein anderes felines Pathogen, umfassend einen ersten *in vivo-*Expressionsvektor nach einem der Ansprüche 7 bis 18 und einen zweiten *in vivo*-Expressionsvektor, in dem eine Sequenz eingeführt ist, die ein Immunogen eines anderen felinen Pathogens codiert.

21. Immunogene Zubereitung oder mehrwertiger Impfstoff gegen das feline Calcivirus und gegen mindestens ein anderes felines Pathogen, umfassend einen *in vivo*-Expressionsvektor nach Anspruch 17 oder 18, und ein aus veterinärer Sicht verträglicher Träger oder Exzipient.

22. Immunogene Zubereitung oder Impfstoff nach einem der Ansprüche 19 bis 21, wobei mindestens einer der *in vivo*-Expressionsvektoren ein Pockenvirus ist.

23. Immunogene Zubereitung oder Impfstoff nach Anspruch 22, wobei der Pockenvirus ein Kanarienpockenvirus ist.

24. Immunogene Zubereitung oder Impfstoff nach einem der Ansprüche 19 bis 23, des Weiteren umfassend ein Adjuvans.

25. Immunogene Zubereitung oder Impfstoff nach Anspruch 24, wobei mindestens einer der *in vivo*-Expressionsvektoren ein Plasmid ist und wobei das Adjuvans ein kationisches Lipid ist, das quarternäres Ammoniumsalz enthält mit der Formel: 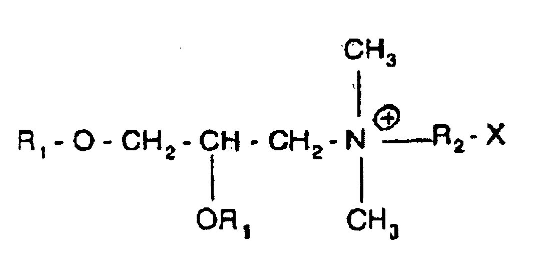 wobei R1 ein lineares, gesättigtes oder ungesättigtes aliphatisches Radikal ist, das 12 bis 18 Kohlenstoffatome hat, R2 ein anderes aliphatisches Radikal ist, das 2 oder 3 Kohlenstoffatome enthält, und X eine Hydroxyl- oder Amingruppe ist.

26. Immunogene Zubereitung oder Impfstoff nach Anspruch 25, wobei das Adjuvans DMRIE ist.

27. Immunogene Zubereitung oder Impfstoff nach einem der Ansprüche 25 und 26, wobei das Adjuvans mit einem neutralen Lipid verknüpft ist.

28. Immunogene Zubereitung oder Impfstoff nach Anspruch 27, wobei das neutrale Lipid DOPE umfasst.

29. Immunogene Zubereitung oder Impfstoff nach Anspruch 27, wobei das neutrale Lipid DMRIE-DOPE umfasst.

30. Immunogene Zubereitung oder Impfstoff nach Anspruch 22 oder 23, der des Weiteren ein Adjuvans umfasst, das ein Polymer einer Acrylsäure oder Methacrylsäure umfasst.

31. Immunogene Zubereitung oder Impfstoff nach Anspruch 24, wobei das Adjuvans ein Carbomer umfasst.

32. Immunogene Zubereitung oder Impfstoff nach einem der Ansprüche 19 bis 31, des Weiteren umfassend einen plasmidischen *in vivo*-Expressionsvektor, in dem ein Gen eingeführt ist, das felines GM-CSF codiert.

33. Immunogene Zubereitung oder Impfstoff gegen das feline Calicivirus, umfassend das Kapsidprotein des Stammes FCV 431, das in Form leerer Kapseln zusammengesetzt ist, wobei das Protein die Sequenz SEQ ID NO:7 hat.

34. Immunogene Zubereitung oder Impfstoff gegen das feline Calicivirus, umfassend ein immunologisch aktives Fragment oder Epitop der Sequenz SEQ ID NO:7, wobei das immunologisch aktive Fragment oder Epitop in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

35. Immunogene Zubereitung oder Impfstoff gegen das feline Calicivirus, umfassend das Kapsidprotein eines felinen Calicivirus, das vom monoclonalen Antikörper 44 erkannt wird, das in Form leerer Kapseln zusammengesetzt ist.

36. Immunogene Zubereitung oder Impfstoff gegen das feline Calicivirus, umfassend ein immunologisch aktives Fragment oder Epitop eines Kapsidproteins eines felinen Calicivirus, das vom monoclonalen Antikörper 44 erkannt wird, wobei das immunologisch aktive Fragment oder Epitop in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

37. Immunogene Zubereitung oder Impfstoff nach einem der Ansprüche 33 bis 36, des Weiteren umfassend ein Kapsidprotein des Stamms FCV G1, das in Form leerer Kapseln zusammengesetzt ist, wobei das Protein die Sequenz SEQ ID NO: 5 hat.

38. Immunogene Zubereitung oder Impfstoff nach einem der Ansprüche 33 bis 36, des Weiteren umfassend ein immunologisch aktives Fragment oder Epitop des Proteins mit der Sequenz SEQ ID NO:5, wobei das immunologisch aktive Fragment oder Epitop in der Lage ist, bei der Katze *in vivo* Antikörper zu induzieren, die das Kreuzneutralisationsspektrum des Antiserums des Stamms FCV G1, wie beim CNCM unter der Hinterlegungsnummer I-2167 hinterlegt, haben.

39. Isoliertes, gereinigtes oder synthetisches Kapsidprotein mit der Sequenz SEQ ID NO:7, das in Form leerer Kapseln zusammengesetzt ist.

40. Immunologisch aktives Fragment oder Epitop des Proteins mit der Sequenz SEQ ID NO:7, wobei das immunologisch aktive Fragment oder Epitop in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

41. Isoliertes, gereinigtes oder synthetisches Kapsidprotein, in Form von leeren Kapseln zusammengesetzt, eines felinen Calicivirus, das vom monoclonalen Antikörper 44 erkannt wird, wobei das Kapsidprotein in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

42. Immunologisch aktives Fragment oder Epitop des isolierten, gereinigten oder synthetischen Kapsidproteins eines felinen Calicivirus, das vom monoclonalen Antikörper 44 erkannt wird, wobei das immunologisch aktive Fragment oder Epitop in der Lage ist, bei der Katze *in vivo* Antikörper zu induzieren, die das Kreuzneutralisationsspektrum des Antiserums des Stamms FCV 431, wie beim CNCM unter der Hinterlegungsnummer I-2166 hinterlegt, haben.

43. Immunogene Zubereitung oder Impfstoff, umfassend ein isoliertes, gereinigtes oder synthetisches Proteinkapsid oder immunologisch aktives Fragment oder Epitop nach einem der Ansprüche 39 bis 42, und ein aus veterinärer Sicht verträglicher Träger oder Exzipient.

44. Immunogene Zubereitung oder Impfstoff nach Anspruch 43, des Weiteren umfassend ein Immunogen eines anderen felinen Pathogens oder einen Expressionsvektor umfassend eine Nucleotidsequenz eines Immunogens eines anderen felinen Pathogens.

45. Immunogene Zubereitung oder Impfstoff nach Anspruch 44, wobei das andere feline Pathogen ausgewählt ist aus der Gruppe bestehend aus feliner viraler Rhinoracheitis (auch felines Herpesvirus, FHV, genannt), Felinem Läukemievirus (FeLV), Felinem Parvovirus (FPV), Felinem Infektiösem Peritonitis Virus (FIPV), Felinem Immundefizienz-Virus (FIV), Tollwut, *Chlamydia.*

46. Immunogene Zubereitung oder Impfstoff nach einem der Ansprüche 43 bis 45, des Weiteren umfassend das isolierte, gereinigte oder synthetische Kapsidprotein mit der Sequenz SEQ ID NO:5.

47. lmmunogene Zubereitung oder Impfstoff nach einem der Ansprüche 43 bis 45, des Weiteren umfassend ein immunologisch aktives Fragment oder Eptiop des Proteins mit der Sequenz SEQ ID NO:5, wobei das immunologisch aktive Fragment oder Eptiop in der Lage ist, bei der Katze *in vivo* Antikörper zu induzieren, die das Kreuzneutralisationsspektrum des Antiserums des Stamms FCV G1, wie beim CNCM unter der Hinterlegungsnummer I-2166 hinterlegt, haben.

48. Immunogene Zubereitung oder Impfstoff nach einem der Ansprüche 43 bis 45, des Weiteren umfassend einen Expressionsvektor, umfassend eine Nucleotidsequenz, die das Protein mit der Sequenz SEQ ID NO:5 codiert.

49. Immunogene Zubereitung oder Impfstoff nach einem der Ansprüche 43 bis 45, des Weiteren umfassend einen Expressionsvektor, umfassend eine Nucleotidsequenz, die ein immunologisch aktives Fragment oder Epitop des Proteins mit der Sequenz SEQ ID NO:5 codiert, wobei das immunologisch aktive Fragment oder Eptiop in der Lage ist, bei der Katze *in vivo* Antikörper zu induzieren, die das Kreuzneutralisationsspektrum des Antiserums des Stamms FCV G1, wie beim CNCM unter der Hinterlegungsnummer I-2167 hinterlegt, haben.
